# EUROPEAN PATENT SPECIFICATION

(11) **EP 2 971 182 B1**
(45) Date of publication and mention of the grant of the patent: **29.06.2022**
(21) Application number: 14778984.6
(22) Date of filing: 12.03.2014
(51) Int. Cl.: C12Q 1/6827

(54) **METHODS FOR PRENATAL GENETIC ANALYSIS**
VERFAHREN ZUR PRÄNATALEN GENETISCHEN ANALYSE
MÉTHODES D'ANALYSE GÉNÉTIQUE PRÉNATALE

(30) Priority: 12.03.2013 US 201361778131 P
(43) Date of publication of application: 20.01.2016
(73) Proprietor: Myriad Women's Health, Inc., South San Francisco, CA 94080 (US)
(72) Inventor: EVANS, Eric, South San Francisco, CA 94080 (US); CHU, Clement, South San Francisco, CA 94080 (US); DAVISON, Daniel, South San Francisco, CA 94080 (US); RICHARDS, Hunter, South San Francisco, CA 94080 (US)
(74) Representative: Hutter, Anton
(86) International application number: PCT/US2014/025031
(87) International publication number: WO 2014/165267

(56) References cited:
- WO-A1-2007/100243
- WO-A2-02/057491
- WO-A2-2009/139929
- WO-A2-2011/142836
- WO-A2-2012/019187
- WO-A2-2012/019187
- US-A1- 2012 295 819
- US-A1- 2013 029 852
- US-A1- 2013 040 375

## Description

### BACKGROUND OF THE DISCLOSURE

In many cases, genetic alterations in a genome contribute to adverse health consequences. Genomics research has identified numerous genes and specific diagnostic markers that are present in abnormal copy numbers, or found mutated, to be associated with a variety of diseases. For example, in prenatal diagnosis, extra or missing copies of whole chromosomes, such as trisomy of chromosome 21, are frequent occurrences and may be detected before a pregnancy develops to term. In other examples, detection of specific mutations, or detection of multiplication or deletion of chromosomes, chromosomal regions or other loci, may be used in the risk assessment, diagnosis, or staging of many cancers.

Methods for detection of genetic variants in a sample are known, for example, from WO 2012/019187. Generally, information about genetic alterations have been assayed using conventional procedures for genetic testing, such as fluorescence in situ hybridization (FISH), quantitative fluorescence PCR (QF-PCR) and array-Comparative Genomic Hybridization (array-CGH) and more recently, next generation sequencing. Next-generation sequencing (NGS) allows small-scale, inexpensive genome sequencing with a turnaround time measured in days. However, as NGS is generally performed and understood, all regions or loci of the genome are sequenced with roughly equal probability, meaning that a large amount of genomic sequence is collected and discarded to collect sequence information from the relatively low percentage of areas where function is understood well enough to interpret potential mutations. Generally, purifying samples of regions one is interested in, from a full-genome, is conducted as a separate step from sequencing. It is usually a days-long, low efficiency process in the current state of the art.

There is a need in the art for improved methods and systems for analyzing genomic sequences of regions or loci of interest that may be associated with potentially adverse genetic alterations.

### SUMMARY OF THE DISCLOSURE

This disclosure generally provides for systems and methods for prenatal genetic analysis. The present invention is defined in the appended claims.

Advantageous embodiments are set out in the appended sub claims. Generally, this disclosure provides for systems and methods of testing for a genetic alteration at one or more loci in a sample comprising a mixture of maternal and fetal DNA polynucleotides, comprising the steps of: obtaining maternal and fetal polynucleotides in a test sample; hybridizing a plurality of probes to at least one locus of interest and to at least one locus outside the locus of interest in the sample comprising maternal and fetal polynucleotides, wherein at least one or more probes is associated with an identifier; optionally extending probes using polymerase; ligating probes to produce a contiguous ligation product; isolating bound ligation products from unbound probes; enumerating one or more regions contained within the ligation product, wherein the one or more regions comprise elements other than fully intact probes and determining the presence or absence of a genetic alteration at one or more loci.

In some aspects, this disclosure provides methods of testing for a genetic alteration at one or more loci in a sample comprising a mixture of maternal and fetal DNA polynucleotides, comprising the steps of: obtaining maternal and fetal polynucleotides in a test sample; hybridizing a plurality of probes to at least one locus of interest and to at least one locus outside the locus of interest in the sample comprising maternal and fetal polynucleotides, wherein at least one or more probes is associated with an identifier; optionally extending probes using polymerase; ligating probes to produce a contiguous ligation product; isolating bound ligation products from unbound probes; amplifying a region from the ligation product; enumerating a region from the ligation product containing the identifier sequence, wherein enumerating comprises enumeration of sequences other than fully intact hybridization sequences; and determining the presence or absence of a genetic alteration at one or more loci.

In some aspects, this disclosure provides methods of testing for a genetic alteration at one or more loci in a sample comprising a mixture of maternal and fetal DNA polynucleotides, comprising the steps of: obtaining maternal and fetal polynucleotides in a test sample; hybridizing to polynucleotides, a plurality of probes comprising hybridization sequences complementary to at least one locus of interest and to at least one locus outside the locus of interest in the sample, comprising maternal and fetal polynucleotides, wherein at least one or more probes is associated with an identifier sequence; hybridizing one or more bridging oligonucleotide to a region between two hybridization sequences in the same locus; optionally extending the probes and/or bridging oligonucleotide(s) using polymerase and dNTPs; ligating the probes and bridging oligonucleotide(s) to produce a contiguous ligation product; isolating contiguous ligation products from unbound probes; amplifying a region from the ligation product containing the identifier sequence and sequences other than fully intact hybridization sequences; enumerating the region amplified in step (g) wherein enumerating comprises enumeration of the identifier sequences and sequences other than fully intact hybridization sequences; and determining the presence or absence of a genetic alteration at one or more loci.

In some aspects, this disclosure provides methods for detecting a genetic alteration at one or more loci in a sample comprising a mixture of maternal and fetal DNA polynucleotides, comprising the steps of: obtaining maternal and fetal polynucleotides in a test sample; hybridizing to polynucleotides, a plurality of probes comprising hybridization sequences complementary to at least one locus of interest and to at least one locus outside the locus of interest in the sample, comprising maternal and fetal polynucleotides, wherein at least one or more probes is associated with an identifier sequence; hybridizing one or more bridging oligonucleotide to a region between two hybridization sequences in the same locus; optionally extending the probes and/or bridging oligonucleotide(s) using polymerase and dNTPs; ligating the probes and bridging oligonucleotide(s) to produce a contiguous ligation product; isolating contiguous ligation products from unbound probes; amplifying a region from the ligation product, wherein the region comprises the identifier and sequences other than fully intact hybridization sequences; enumerating the identifier sequence; and determining the presence or absence of a genetic alteration at one or more loci.

In some aspects, this disclosure provides for a method or assay system for the determining of the presence or absence of a genetic alteration of a locus in a sample comprising a mixture of fetal and maternal polynucleotides, wherein the assay system comprises the enumeration of a identifier sequence associated with a probe contacted to a locus in the sample.

In some aspects, this disclosure provides for a composition of matter, wherein said composition is tested according to the methods as described herein.

In some embodiments, a genetic alteration is a copy number variation (CNV). In some embodiments, a genetic alteration is a causal variant.

In some embodiments, the identifier or identifier is a barcode sequence. In some aspects, probes are separate fixed sequences complementary to regions in one or more loci.

In some embodiments, hybridization sequences are separate fixed sequences complementary to regions in one or more loci. In some aspects, probes comprise pre-circle probes with sequences complementary to regions in one or more loci.

In some embodiments, selectively amplifying a region from the ligation product comprises one or more amplification steps. In some aspects, enumerating comprises a sequencing step.

In some embodiments, intact hybridization sequences comprise no hybridization sequences. In some embodiments, intact hybridization sequences comprises less than 100% of hybridization sequences complementary to a locus. In some embodiments, enumerating comprises enumerating sequences not containing hybridization sequences.

In some embodiments, amplifying is performed through a universal amplification step. In some embodiments, amplifying is performed through a selective amplification step. In some embodiments, amplifying is performed on sequences not containing hybridization sequences.

In some aspects, at least one locus is tested for a genetic alteration. In some aspects, at least 100 loci are tested for genetic alterations. In some aspects, at least 500 loci are tested for genetic alterations. In some aspects, at least 1000 loci are tested for genetic alterations. In some aspects, at least one locus contains a polymorphism or putative polymorphism.

In some aspects, no amplification step is used.

In some aspects, at least one locus is tested for copy number and is different than another locus containing a polymorphism. In some aspects, the locus is a chromosome, a sub-chromosomal region, or a single locus.

In some embodiments, at least one bridging oligonucleotide hybridizes to a region between two probes.

In some embodiments, isolating contiguous ligation products comprises degradation of unbound probes. In some embodiments, degradation is performed using an exonuclease.

In some embodiments, isolating contiguous ligation products comprises affinity capture with a binding partner. In some aspects, dNTPs are conjugated to a moiety for affinity capture. In some aspects, dNTPs are conjugated to biotin.

In some embodiments, the genetic alteration is fetal aneuploidy.

In some embodiments a medical decision or treatment recommendation is made based on determining the presence or absence of a genetic alteration.

In some embodiments, the enumerating step is performed using statistical analysis or is performed by a computer readable medium having processor-executable instructions. In some aspects, statistical analysis may be performed using a computer algorithm.

In one aspect, the disclosure provides a method of testing for a genetic alteration at one or more loci in a sample comprising a mixture of maternal and fetal DNA polynucleotides. In one embodiment, the method comprises the steps of: fragmenting target polynucleotides; joining adapter oligonucleotides to the fragmented polynucleotides; amplifying adapted polynucleotides using amplification primers that specifically hybridize to sequences derived from the adapter oligonucleotides; hybridizing amplified target polynucleotides to a plurality of different bound oligonucleotides attached to a solid surface, wherein each of a plurality of the bound oligonucleotides comprise a 3'-end sequence that is complementary to a sequence comprising a locus of interest, or a sequence within 200 nucleotides of a locus of interest; performing bridge amplification on the solid support to specifically amplify target sequences; and determining the presence or absence of a genetic alteration at one or more loci.

### BRIEF DESCRIPTION OF THE DRAWINGS

The novel features of this disclosure are set forth with particularity in the appended claims. A better understanding of the features and advantages of this disclosure will be obtained by reference to the following detailed description that sets forth illustrative embodiments, in which the principles of this disclosure are utilized, and the accompanying drawings of which:
**Fig. 1** is a schematic diagram of an example flow scheme for CNV detection at one or more loci.
**Fig. 2** is a schematic diagram of an example flow scheme for CNV detection of one or more chromosomes using MIP probes.
**Fig. 3** is a schematic diagram of an example flow scheme for CNV detection of one or more loci using MIP probes using detection of SNPs and allele frequency.
**Fig. 4** is a schematic representation of a MIP probe bound to genomic DNA, ligation of the hybridization sequences and second strand synthesis.

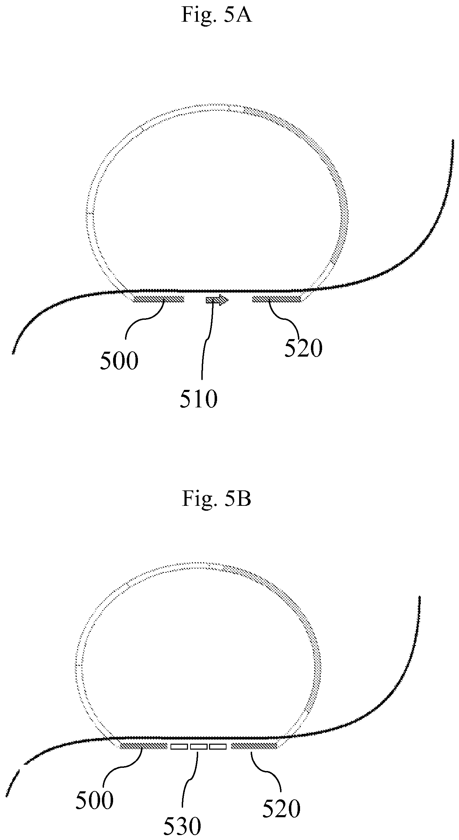

**Fig. 5A** is a schematic representation of a MIP probe bound to genomic DNA with a bridging probe hybridized in a region between two probes.
**Fig. 5B** is a schematic representation of a MIP probe bound to genomic DNA with a gap between two probes. Polymerase i is used in a primer extension step to fill the gap between probes.
**Fig. 6A** is a schematic representation of a configuration of hybridization sequences, **600** and **650,** universal primer sites, **610** and **620,** barcode sequence, **660** and restriction site, **699** in a MIP probe (circular linker region not shown). After hybridization and enzymatic cleavage at site **699,** universal primer sites flank hybridization sequences and the barcode sequence. Example priming sites for optional amplification or sequencing are also shown.
**Fig. 6B** is a schematic representation similar to **Fig. 6A****,** showing identical elements in an alternative configuration of a MIP probe (circular linker region not shown). After hybridization and enzymatic cleavage at site **699,** universal primer sites flank the barcode sequence. Example priming sites for optional amplification or sequencing are also shown.
**Fig. 7** is a schematic representation of double stranded circular contiguous ligation product that may generated as shown in **Fig. 4** from a linear MIP probe. Arrows indicate various priming sites for either amplification or sequencing of various regions, or combinations of regions, from this product.
**Fig. 8** is a schematic representation and flow diagram of a computer with non-transmissible storage medium used for storage and dissemination of genomic or sequencing information using the internet. Sequencing and generation of genomic data may be performed at a site different than the physical location of the user.
**Fig. 9** illustrates an example probe, an example pair of amplification primers, and circularization of the probe.
**Fig. 10** illustrates example primer extension steps to amplify a target probe sequence.
**Fig. 11** illustrates a portion of an example solid support comprising attached oligonucleotides, and the first steps in an example bridge amplification process to amplify a target polynucleotide.
**Fig. 12** illustrates an example process of target amplification, bridge amplification, and sequencing.

### DETAILED DESCRIPTION OF THE DISCLOSURE

### I. General Terminology

The systems and methods of this disclosure as described herein may employ, unless otherwise indicated, conventional techniques and descriptions of molecular biology (including recombinant techniques), cell biology, biochemistry, microarray and sequencing technology, which are within the skill of those who practice in the art. Such conventional techniques include polymer array synthesis, hybridization and ligation of oligonucleotides, sequencing of oligonucleotides, and detection of hybridization using a label. Specific illustrations of suitable techniques can be had by reference to the examples herein. However, equivalent conventional procedures can, of course, also be used. Such conventional techniques and descriptions can be found in standard laboratory manuals such as Green, et al., Eds., Genome Analysis: A Laboratory Manual Series (Vols. I-IV) (1999); Weiner, et al., Eds., Genetic Variation: A Laboratory Manual (2007); Dieffenbach, Dveksler, Eds., PCR Primer: A Laboratory Manual (2003); Bowtell and Sambrook, DNA Microarrays: A Molecular Cloning Manual (2003); Mount, Bioinformatics: Sequence and Genome Analysis (2004); Sambrook and Russell, Condensed Protocols from Molecular Cloning: A Laboratory Manual (2006); and Sambrook and Russell, Molecular Cloning: A Laboratory Manual (2002) (all from Cold Spring Harbor Laboratory Press); Stryer, L., Biochemistry (4th Ed.) W.H. Freeman, N.Y. (1995); Gait, "Oligonucleotide Synthesis: A Practical Approach" IRL Press, London (1984); Nelson and Cox, Lehninger, Principles of Biochemistry, 3rd Ed., W.H. Freeman Pub., New York (2000); and Berg et al., Biochemistry, 5th Ed., W.H. Freeman Pub., New York (2002). Before the present compositions, research tools and systems and methods are described, it is to be understood that this disclosure is not limited to the specific systems and methods, compositions, targets and uses described, as such may, of course, vary. It is also to be understood that the terminology used herein is for the purpose of describing particular aspects only and is not intended to limit the scope of the present disclosure, which will be limited only by appended claims.

The terminology used therein is for the purpose of describing particular embodiments only and is not intended to be limiting of a device of this disclosure. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

Several aspects of a systems and methods of this disclosure are described above with reference to example applications for illustration. It should be understood that numerous specific details, relationships, and systems and methods are set forth to provide a full understanding of the disclosure. One having ordinary skill in the relevant art, however, will readily recognize that the systems and methods of this disclosure can be practiced without one or more of the specific details or with other systems and methods. This disclosure is not limited by the illustrated ordering of acts or events, as some acts may occur in different orders and/or concurrently with other acts or events. Furthermore, not all illustrated acts or events are required to implement a methodology in accordance with this disclosure.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. The term "about" as used herein refers to a range that is 15% plus or minus from a stated numerical value within the context of the particular usage. For example, about 10 would include a range from 8.5 to 11.5.

### II. Overview

The present disclosure provides for systems and methods for the testing and analysis of genetic alterations of a sample comprising maternal and fetal polynucleotides. Generally, the systems and methods of this disclosure provide for the isolation of a mixture of maternal and fetal polynucleotides from a sample, generally from the mother. Polynucleotides are isolated and purified and further tested to determine the presence or absence of genetic alterations, such as copy number variation, or causal variants at one or more loci in the sample.

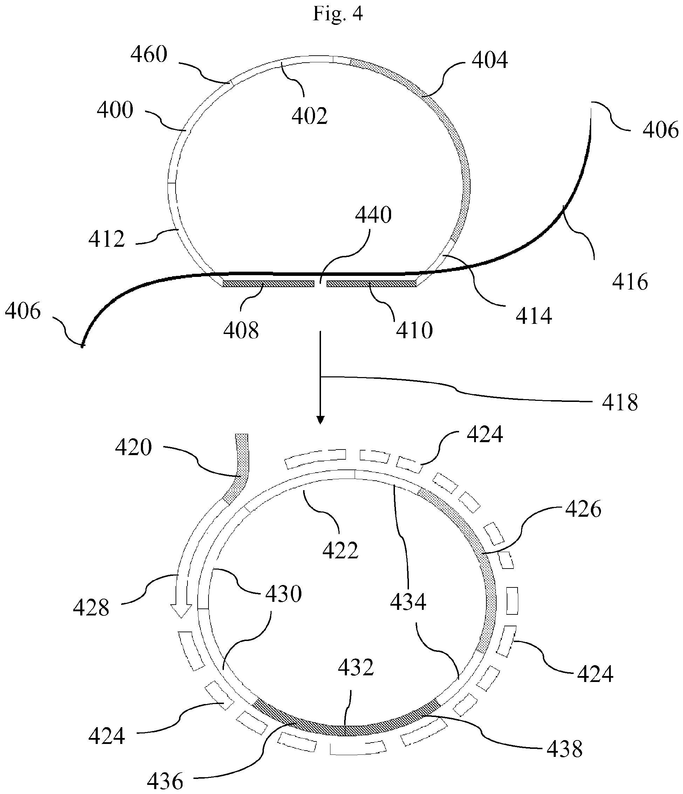

Generally, as shown in **Fig. 4****,** one or more probes sets, each comprising at least two hybridization sequences, **408** and **410,** complementary for sequences in the sample samplestrand, **416,** are hybridized to one or more loci of interest in the sample and one or more loci outside the loci of interest. Probes are generally assigned an identifier molecule such as a molecular barcode sequence, **404.** In some cases, a probe set may comprise a molecular inversion probe (MIP), **400,** and constructed from additional sequences, such as universal primer sequences, **402** and **414,** a restriction site, **460,** and linker sequences, **412.** Two or more probes are then ligated, **418,** to produce a single contiguous ligation product at the ligation site, **432.** Bound probes may be isolated through treatment with exonuclease, which selectively targets ends of linear polynucleotides, **406** and may digest sample DNA strands and unbound probes while leaving bound circular probes intact. Identifier molecules are identified and enumerated using various means, including universal amplification from universal priming sites, **434,** and **430,** sites in the barcode sequence, **426,** or sites in the hybridization sequences, **438** and **436.** Generally, the abundance of barcodes, previously assigned to particular probe set, is generally proportional to the abundance of loci to which the particular probe set is complementary. Thus, the abundance of barcodes may be proportional to the copy number of the loci originally present in the sample. The system and methods of this disclosure provide for various methods for enumeration of identifiers and the use of enumerated identifiers in determining the presence or absence of genetic alterations in a sample, such as determining CNV at one or more loci.

Further, the systems and methods of this disclosure may be particularly useful to detect a variety of genetic alterations in maternal and fetal samples, ranging from whole chromosomal abnormalities to other subtle genomic alterations that may be indicative other conditions. For example, the systems and methods of this disclosure may be useful in detecting trisomy 21 or providing detection of causal variants for diseases such as cystic fibrosis.

### III. Polynucleotide Isolation and Extraction

### A. Sources of Test Sample

The systems and methods of this disclosure may involve the testing, manipulation, preparation, identification and/or quantification of a variety of polynucleotides. Examples of polynucleotides include but are not limited to: DNA, RNA, amplicons, cDNA, dsDNA, ssDNA, plasmid DNA, cosmid DNA, high Molecular Weight (MW) DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA (e.g., retroviral RNA).

The test sample may be derived from any suitable biological source, comprising a mixture of maternal and fetal polynucleotides. Samples may be derived from a variety of sources including human, mammal, non-human mammal, ape, monkey, chimpanzee, reptilian, amphibian, or avian, sources. In some cases, a test sample may comprise a tissue sample, a biological fluid, or a cell sample. In some cases, a test sample may comprise a swab, smear, a biopsy specimen, aspiration, or a plurality of cells cultured in vitro (i.e. tissue/cell culture). In some cases, polynucleotides may be extracted from cells and subsequently tested. In other cases, polynucleotides may be found as cell-free, such as polynucleotides circulating in bodily fluids, such as blood, and subsequently tested. Examples of biological sources may include but are not limited to maternal organ tissue, blood, plasma, serum, sweat, tears, sputum, urine, ear secretions, lymph, saliva, cerebrospinal fluid, bone, bone marrow suspension, vaginal secretions, transcervical lavage, brain fluid, leukophoresis samples, ascites, milk, respiratory secretions, mucus, intestinal secretions, amniotic fluid, chorionic villi samples, umbilical cord samples, or placental samples.

In some cases, samples can be obtained from a single source or combination of sources. In some cases, a mixture of maternal and fetal polynucleotides may be obtained from the same biological sample. In some cases, a mixture of maternal and fetal polynucleotides may be obtained from a single maternal sample, such as maternal blood. In some cases, samples may be obtained from separate samples. In some cases maternal and fetal polynucleotides may be mixed after sampling. In some cases, a sample may be derived from different individuals, different developmental stages of the same or different individuals, individuals with differing disease states (i.e. individuals with cancer, or a suspected genetic disorder), normal or healthy individuals, individuals in one or more disease states, individuals subjected to different treatments for disease, individuals exposed to different environmental factors or individuals exposed to different infectious or disease agent (i.e. virus, bacteria, pathogen). Generally, a biological sample may be derived from a pregnant female.

Samples may also be obtained from cells in cell culture, such as in vitro cultured tissues, cultured cells or other cultured polynucleotide containing sources. Samples taken from in vitro sources may be cultured under a variety of conditions, including but not limited to different types of media conditions (i.e. pH, temperature, growth factors, nutritional components, etc ...), length of culture time, or treatment with exogenous factors (i.e. drugs, drugs candidates, chemical agents, toxins, etc... ).

After a sample has been obtained, polynucleotides may be extracted, isolated and purified using any suitable techniques known in the art. For example, in some cases, DNA may be isolated, extracted and prepared using any suitable commercially available kits, such as the Qiagen Qiamp^{®} Circulating Nucleic Acid Kit. Other examples include but are not limited to Qiagen Qubit^{™} dsDNA HS Assay kit protocol, Agilent^{™} DNA 1000 kit, or TruSeq^{™} Sequencing Library Preparation; Low-Throughput (LT) or other kits provided by companies such as Sigma Aldrich, Life Technologies, Promega, Affymetrix, IBI or the like. Any suitable non-commercial kits may also be used for isolation and purification of polynucleotides for the systems and methods of this disclosure.

After purification, in some cases, polynucleotides may be pre-mixed with one or more additional materials, such as one or more reagents. Reagents, may include but are not limited to ligase, protease, polymerase, restriction enzymes, dNTPs, salts, bulk polynucleotides and the like. One or more reagents may be added for preparation of polynucleotides before subsequent hybridization steps.

### B. Polynucleotide Fragmentation

After isolation and purification, systems and methods of the disclosure provide for optional preparation steps for fragmenting polynucleotides before hybridization. In some cases, polynucleotides may be isolated and purified as fragments, such as in cases where polynucleotides are degraded or found as short sequences (e.g. cell free polynucleotides). In other cases, polynucleotides may be isolated and purified as intact or substantially intact sequences, such as genomic DNA (gDNA).

Fragmentation of sample strands, such as gDNA, may be particularly useful in some cases. For example, polynucleotides may be partitioned before hybridization, wherein single strands or fragments are separated and partitioned, such as in a single well, single drop or single emulsion. gDNA may be fragmented into non-overlapping sequences, which may be partitioned and combined with various other reagents inside the partitions. In one example, barcode sequences, as herein described, may be assigned to probe sequences within partitions.

Partitioning may be performed with any suitable methods or devices. For example, microfluidic devices are known for distribution of a sample or reaction mixture followed by addition of reagents. Various commercial platforms used for distribution and combinatorial addition of reagents may be used with the systems and methods of this disclosure. For example, the Dynamic Array^{™} and Access Array^{™} systems, and as well as systems described in the literature may be used (see, e.g., U.S. Pat. No. 7,604,965; Patent publications WO 2010/077618; US 2009/0317798; US 2008/0223721; US 2009/0257920; US 2009/0291435; US2011/0126910 and WO2011066588. Other approaches include use of microfluidic cards. One useful approach involves distribution of the reaction mixture into microdropletes in which amplification reactions may be carried out (see, e.g., Patent Application Publication Nos. US 2009/0035838; US 2010/0022414; WO 01/89788; WO 2006/040551; WO 2006/040554; WO 2004/002627; WO 2008/063227; WO 2004/091763; WO 2005/021151; WO 2006/096571; WO 2007/089541; WO 2007/081385 and WO 2008/063227. In one droplet-based approach the sample may be partitioned into a plurality of droplets and individual same droplets fused with droplets containing specified reagents.

In some cases polynucleotides may be fragmented into sizes about 10-50, 50-100, 100-500, 500-1000, 1000-3000, or 1000-3000 base pairs in length. In some cases polynucleotides may be fragmented into sizes at least 10, 20, 30, 40, 50, 60, 70, 80, 90, 100, 200, 300, 400, 500 , 600, 700, 800, 900, 1000, 2000, 3000, or 5000 base pairs in length. In some cases polynucleotides may be fragmented into sizes at most 10, 20, 30, 40, 50, 60, 70, 80, 90, 100, 200, 300, 400, 500 , 600, 700, 800, 900, 1000, 2000, 3000, or 5000 base pairs in length.

Numerous fragmentation systems and methods are described herein and known in the art. For example, fragmentation may be performed through physical, mechanical or enzymatic systems and methods. Physical fragmentation may include exposing a target polynucleotide to heat or to UV light. Mechanical disruption may be used to mechanically shear a target polynucleotide into fragments of the desired range such as through sonication or nebulization. Target polynucleotides may also be fragmented using enzymatic systems and methods. In some cases, enzymatic digestion may be performed using enzymes such as using restriction enzymes.

Restriction enzymes may be used to perform specific or non-specific fragmentation of target polynucleotides. The systems and methods of the present disclosure may use one or more types of restriction enzymes, generally described as Type I enzymes, Type II enzymes, and/or Type III enzymes. Type II and Type III enzymes are generally commercially available and well known in the art.

### IV. Target Amplification and Sequencing

A method for sequencing a plurality of target polynucleotides in a sample is disclosed. In one embodiment, the method comprises: (a) fragmenting target polynucleotides to produce fragmented polynucleotides; (b) joining adapter oligonucleotides to the fragmented polynucleotides, each of the adapter oligonucleotides comprising sequence D, to produce adapted polynucleotides comprising sequence D hybridized to complementary sequence D' at both ends of the adapted polynucleotides, optionally wherein sequence D' is produced by extension of a target polynucleotide 3' end; (c) amplifying the adapted polynucleotides using amplification primers comprising sequence C, sequence D, and a barcode associated with the sample, wherein sequence D is positioned at the 3' end of the amplification primers; (d) hybridizing amplified target polynucleotides to a plurality of different first oligonucleotides that are attached to a solid surface; (e) performing bridge amplification on a solid surface; and (f) sequencing a plurality of polynucleotides from step (e). The solid surface may comprise a plurality of oligonucleotides. In some embodiments, the solid surface comprises (i) a plurality of different first oligonucleotides comprising sequence A and sequence B, wherein sequence A is common among all first oligonucleotides; and further wherein sequence B is different for each different first oligonucleotide, is at the 3' end of each first oligonucleotide, and is complementary to a sequence comprising a locus of interest or a sequence within 200 nucleotides of a locus of interest; (ii) a plurality of second oligonucleotides comprising sequence A at each 3' end; and (iii) a plurality of third oligonucleotides comprising sequence C at each 3' end. In some embodiments, one or more of sequences A, B, C, and D are different sequences. In some embodiments, one or more of sequences A, B, C, and D are about, less than about, or more than about 5%, 10%, 15%, 20%, 25%, 30%, 40%, 50%, 60%, 70%, 80%, 90%, or more different from one or more of the other of sequences A, B, C, and D (e.g. have less than about 10%, 20%, 30%, 40%, 50%, 60%, 70%, 80%, 90%, or more sequence identity). In some embodiments, one or more of sequences A, B, C, and D comprise about, less than about, or more than about 1, 2, 3, 4, 5, 6, 7, 8, 9, 10, 15, 20, or more nucleotides each.

Samples may be obtained and fragmented by an appropriate method, such as a method described herein. In some embodiments, fragmentation is followed by ligation of adapter oligonucleotides to the fragmented polynucleotides. An adapter oligonucleotide includes any oligonucleotide having a sequence, at least a portion of which is known, that can be joined to a target polynucleotide. Adapter oligonucleotides can comprise DNA, RNA, nucleotide analogues, non-canonical nucleotides, labeled nucleotides, modified nucleotides, or combinations thereof. Adapter oligonucleotides can be single-stranded, double-stranded, or partial duplex. In general, a partial-duplex adapter comprises one or more single-stranded regions and one or more double-stranded regions. Double-stranded adapters can comprise two separate oligonucleotides hybridized to one another (also referred to as an "oligonucleotide duplex"), and hybridization may leave one or more blunt ends, one or more 3' overhangs, one or more 5' overhangs, one or more bulges resulting from mismatched and/or unpaired nucleotides, or any combination of these. In some embodiments, a single-stranded adapter comprises two or more sequences that are able to hybridize with one another. When two such hybridizable sequences are contained in a single-stranded adapter, hybridization yields a hairpin structure (hairpin adapter). When two hybridized regions of an adapter are separated from one another by a non-hybridized region, a "bubble" structure results. Adapters comprising a bubble structure can consist of a single adapter oligonucleotide comprising internal hybridizations, or may comprise two or more adapter oligonucleotides hybridized to one another. Internal sequence hybridization, such as between two hybridizable sequences in an adapter, can produce a double-stranded structure in a single-stranded adapter oligonucleotide. Adapters of different kinds can be used in combination, such as a hairpin adapter and a double-stranded adapter, or adapters of different sequences. Different adapters can be joined to target polynucleotides in sequential reactions or simultaneously. In some embodiments, identical adapters are added to both ends of a target polynucleotide. For example, first and second adapters can be added to the same reaction. Adapters can be manipulated prior to combining with target polynucleotides. For example, terminal phosphates can be added or removed.

In some embodiments, an adapter is a mismatched adapter formed by annealing two partially complementary polynucleotide strands so as to provide, when the two strands are annealed, at least one double-stranded region and at least one unmatched region. The "double-stranded region" of the adapter is a short double-stranded region, typically comprising 5 or more consecutive base pairs, formed by annealing of the two partially complementary polynucleotide strands. This term simply refers to a double-stranded region of nucleic acid in which the two strands are annealed and does not imply any particular structural conformation. In some embodiments, the double-stranded region is about, less than about, or more than about 5, 10, 15, 20, 25, 30, or more nucleotides in length. Generally it is advantageous for the double-stranded region of a mismatched adapter to be as short as possible without loss of function. By "function" in this context is meant that the double-stranded region form a stable duplex under standard reaction conditions for an enzyme-catalyzed nucleic acid ligation reaction, which conditions are known to those skilled in the art (e.g. incubation at a temperature in the range of from 4° C. to 25° C. in a ligation buffer appropriate for the enzyme), such that the two strands forming the adapter remain partially annealed during ligation of the adapter to a target molecule. It is not absolutely necessary for the double-stranded region to be stable under the conditions typically used in the annealing steps of primer extension or PCR reactions. Typically, the double-stranded region is adjacent to the "ligatable" end of the adapter, i.e. the end that is joined to a target polynucleotide in a ligation reaction. The ligatable end of the adapter may be blunt or, in other embodiments, short 5' or 3' overhangs of one or more nucleotides may be present to facilitate/promote ligation. The 5' terminal nucleotide at the ligatable end of the adapter is typically phosphorylated to enable phosphodiester linkage to a 3' hydroxyl group on a sample polynucleotide. The term "unmatched region" refers to a region of the adapter wherein the sequences of the two polynucleotide strands forming the adapter exhibit a degree of non-complementarity such that the two strands are not capable of annealing to each other under standard annealing conditions for a primer extension or PCR reaction. The two strands in the unmatched region may exhibit some degree of annealing under standard reaction conditions for an enzyme-catalyzed ligation reaction, provided that the two strands revert to single stranded form under annealing conditions.

Adapter oligonucleotides can contain one or more of a variety of sequence elements, including but not limited to, one or more amplification primer annealing sequences or complements thereof, one or more sequencing primer annealing sequences or complements thereof, one or more barcode sequences, one or more common sequences shared among multiple different adapters or subsets of different adapters, one or more restriction enzyme recognition sites, one or more overhangs complementary to one or more target polynucleotide overhangs, one or more probe binding sites (e.g. for attachment to a sequencing platform, such as a flow cell for massive parallel sequencing, such as an apparatus as described herein, or flow cells as developed by Illumina, Inc.), one or more random or near-random sequences (e.g. one or more nucleotides selected at random from a set of two or more different nucleotides at one or more positions, with each of the different nucleotides selected at one or more positions represented in a pool of adapters comprising the random sequence), and combinations thereof. Two or more sequence elements can be non-adjacent to one another (e.g. separated by one or more nucleotides), adjacent to one another, partially overlapping, or completely overlapping. For example, an amplification primer annealing sequence can also serve as a sequencing primer annealing sequence. Sequence elements can be located at or near the 3' end, at or near the 5' end, or in the interior of the adapter oligonucleotide. When an adapter oligonucleotide is capable of forming secondary structure, such as a hairpin, sequence elements can be located partially or completely outside the secondary structure, partially or completely inside the secondary structure, or in between sequences participating in the secondary structure. A sequence element may be of any suitable length, such as about, less than about, or more than about 3, 4, 5, 6, 7, 8, 9, 10, 15, 20, 25, 30, 35, 40, 45, 50 or more nucleotides in length. Adapter oligonucleotides can have any suitable length, at least sufficient to accommodate the one or more sequence elements of which they are comprised. In some embodiments, adapters are about, less than about, or more than about 10, 15, 20, 25, 30, 35, 40, 45, 50, 55, 60, 65, 70, 75, 80, 90, 100, 200, or more nucleotides in length

In some embodiments, the adapter oligonucleotides joined to fragmented polynucleotides from one sample comprise one or more sequences common to all adapter oligonucleotides and a barcode that is unique to the adapters joined to polynucleotides of that particular sample, such that the barcode sequence can be used to distinguish polynucleotides originating from one sample or adapter joining reaction from polynucleotides originating from another sample or adapter joining reaction. In some embodiments, an adapter oligonucleotide comprises a 5' overhang, a 3' overhang, or both that is complementary to one or more target polynucleotide overhangs. Complementary overhangs can be one or more nucleotides in length, including but not limited to 1, 2, 3, 4, 5, 6, 7, 8, 9, 10, 11, 12, 13, 14, 15, or more nucleotides in length. Complementary overhangs may comprise a fixed sequence. Complementary overhangs of an adapter oligonucleotide may comprise a random sequence of one or more nucleotides, such that one or more nucleotides are selected at random from a set of two or more different nucleotides at one or more positions, with each of the different nucleotides selected at one or more positions represented in a pool of adapters with complementary overhangs comprising the random sequence. In some embodiments, an adapter overhang is complementary to a target polynucleotide overhang produced by restriction endonuclease digestion. In some embodiments, an adapter overhang consists of an adenine or a thymine.

In some embodiments, adapter oligonucleotides comprise one strand comprising the sequence element sequence D. In some embodiments, adapter oligonucleotides comprise sequence D hybridized to complementary sequence D', where sequence D' is on the same or different strand as sequence D. In some embodiments, the 3' end of a target polynucleotide is extended along an adapter oligonucleotide to generate complementary sequence D'. In some embodiments, fragmented polynucleotides and adapter oligonucleotides are combined and treated (e.g. by ligation and optionally by fragment extension) to produce double-stranded, adapted polynucleotides comprising fragmented polynucleotide sequence joined to adapter oligonucleotide sequences at both ends, where both ends of the adapted polynucleotides comprise sequence D hybridized to sequence D'. In some embodiments, the amount of fragmented polynucleotides subjected to adapter joining is about, less than about, or more than about 50ng, 100ng, 200ng, 300ng ,400ng, 500ng, 600ng, 700ng, 800ng,900ng, 1000ng, 1500ng, 2000ng, 2500ng, 5000ng, 10µg, or more (e.g. a threshold amount). In some embodiments, the amount of fragmented polynucleotides is determined before proceeding with adapter joining, where adapter joining is not performed if the amount is below a threshold amount.

The terms "joining" and "ligation" as used herein, with respect to two polynucleotides, such as an adapter oligonucleotide and a sample polynucleotide, refer to the covalent attachment of two separate polynucleotides to produce a single larger polynucleotide with a contiguous backbone. Non-limiting examples of methods for joining two polynucleotides include enzymatic and non-enzymatic (e.g. chemical) methods. Examples of ligation reactions that are non-enzymatic include the non-enzymatic ligation techniques described in U.S. Pat. Nos. 5,780,613 and 5,476,930. In some embodiments, an adapter oligonucleotide is joined to a fragmented polynucleotide by a ligase, for example a DNA ligase or RNA ligase. Non-limiting examples of ligases, each having characterized reaction conditions, include NAD⁺-dependent ligases including tRNA ligase, Taq DNA ligase, *Thermus filiformis* DNA ligase, *Escherichia coli* DNA ligase, Tth DNA ligase, *Thermus scotoductus* DNA ligase (I and II), thermostable ligase, Ampligase thermostable DNA ligase, VanC-type ligase, 9° N DNA Ligase, Tsp DNA ligase, and novel ligases discovered by bioprospecting; ATP-dependent ligases including T4 RNA ligase, T4 DNA ligase, T3 DNA ligase, T7 DNA ligase, Pfu DNA ligase, DNA ligase 1, DNA ligase III, DNA ligase IV, and novel ligases discovered by bioprospecting; and wild-type, mutant isoforms, and genetically engineered variants thereof. Ligation can be between polynucleotides having hybridizable sequences, such as complementary overhangs. Ligation can also be between two blunt ends. Generally, a 5' phosphate is utilized in a ligation reaction. The 5' phosphate can be provided by the fragmented polynucleotide, the adapter oligonucleotide, or both. 5' phosphates can be added to or removed from polynucleotides to be joined, as needed. Methods for the addition or removal of 5' phosphates are known in the art, and include without limitation enzymatic and chemical processes. Enzymes useful in the addition and/or removal of 5' phosphates include kinases, phosphatases, and polymerases. In some embodiments, both of the two ends joined in a ligation reaction (e.g. an adapter end and a fragmented polynucleotide end) provide a 5' phosphate, such that two covalent linkages are made in joining the two ends, at one or both ends of a fragmented polynucleotide. In some embodiments, 3' phosphates are removed prior to ligation. In some embodiments, an adapter oligonucleotide is added to both ends of a fragmented polynucleotide, wherein one or both strands at each end are joined to one or more adapter oligonucleotides. In some embodiments, separate ligation reactions are carried out for different samples using a different adapter oligonucleotide comprising at least one different barcode sequence for each sample, such that no barcode sequence is joined to the target polynucleotides of more than one sample to be analyzed in parallel.

Non-limiting examples of adapter oligonucleotides include the double-stranded adapter formed by hybridizing CACTCAGCAGCACGACGATCACAGATGTGTATAAGAGACAGT (SEQ ID NO: 17) to GTGAGTCGTCGTGCTGCTAGTGTCTACACATATTCTCTGTC (SEQ ID NO: 18). Additional non-limiting examples of adapter oligonucleotides are described in US20110319290 and US20070128624.

In some embodiments, adapted polynucleotides are subjected to an amplification reaction that amplifies target polynucleotides in the sample. In some embodiments, amplification uses primers comprising sequence C, sequence D, and a barcode associated with the sample, wherein sequence D is positioned at the 3' end of the amplification primers. Amplification primers may be of any suitable length, such as about, less than about, or more than about 5, 10, 15, 20, 25, 30, 35, 40, 45, 50, 55, 60, 65, 70, 75, 80, 90, 100, or more nucleotides, any portion or all of which may be complementary to the corresponding target sequence to which the primer hybridizes (e.g. about, less than about, or more than about 5, 10, 15, 20, 25, 30, 35, 40, 45, 50, or more nucleotides). "Amplification" refers to any process by which the copy number of a target sequence is increased. Any appropriate method for primer-directed amplification of target polynucleotides may be used, examples of which include without limitation, methods based on the polymerase chain reaction (PCR). Conditions favorable to the amplification of target sequences by PCR can be optimized at a variety of steps in the process, and may depend on characteristics of elements in the reaction, such as target type, target concentration, sequence length to be amplified, sequence of the target and/or one or more primers, primer length, primer concentration, polymerase used, reaction volume, ratio of one or more elements to one or more other elements, and others, some or all of which can be altered. In general, PCR involves the steps of denaturation of the target to be amplified (if double stranded), hybridization of one or more primers to the target, and extension of the primers by a DNA polymerase, with the steps repeated (or "cycled") in order to amplify the target sequence. Steps in this process can be optimized for various outcomes, such as to enhance yield, decrease the formation of spurious products, and/or increase or decrease specificity of primer annealing. Methods of optimization include adjustments to the type or amount of elements in the amplification reaction and/or to the conditions of a given step in the process, such as temperature at a particular step, duration of a particular step, and/or number of cycles. In some embodiments, an amplification reaction comprises at least 5, 10, 15, 20, 25, 30, 35, 50, or more cycles. In some embodiments, an amplification reaction comprises no more than 5, 10, 15, 20, 25, 35, 50, or more cycles. Cycles can contain any number of steps, such as 1,2, 3, 4, 5, 6, 7, 8, 9, 10 or more steps. Steps can comprise any temperature or gradient of temperatures, suitable for achieving the purpose of the given step, including but not limited to, strand denaturation, primer annealing, and primer extension. Steps can be of any duration, including but not limited to about, less than about, or more than about 1, 5, 10, 15, 20, 25, 30, 35, 40, 45, 50, 55, 60, 70, 80, 90, 100, 120, 180, 240, 300, 360, 420, 480, 540, 600, or more seconds, including indefinitely until manually interrupted. Cycles of any number comprising different steps can be combined in any order.

In some embodiments, amplification comprises hybridization between sequence D at the 3' end of an amplification primer and sequence D' of an adapted polynucleotide, extension of the amplification primer along the adapted polynucleotide to produce a primer extension product comprising sequence D derived from the amplification primer and sequence D' produced during primer extension. In some embodiments, the amplification process is repeated one or more times by denaturing the primer extension product from a template polynucleotide, and repeating the process using the primer extension product as template for further primer extension reactions. In some embodiments, the first cycle of primer extension is repeated using the same primer as the primer used in the first primer extension reaction, such as for about, less than about, or more than about 5, 10, 15, 20, 25, 30, 35, 50, or more cycles. In some embodiments, one or more primer extensions by the amplification primer is followed by one or more amplification cycles using a second amplification primer having a 3' end comprising a sequence complementary to a sequence added to the adapted polynucleotides by amplification with the first amplification primer (e.g. complementary to the complement of sequence C, or a portion thereof). In some embodiments, the second amplification primer comprises sequence C, or a portion thereof, at the 3' end. A non-limiting example of a second amplification primer includes CGAGATCTACACGCCTCCCTCGCGCCATCAG (SEQ ID NO: 19). In some embodiments, amplification by the second amplification primer comprises about, less than about, or more than about 5, 10, 15, 20, 25, 30, 35, 50, or more cycles. In some embodiments, the amount of adapted polynucleotides subjected to amplification is about, less than about, or more than about 50ng, 100ng, 200ng, 300ng, 400ng, 500ng, 600ng, 700ng, 800ng, 900ng, 1000ng, 1500ng, 2000ng, 2500ng, 5000ng, 10µg, or more (e.g. a threshold amount). In some embodiments, the amount of adapted polynucleotides is determined before proceeding with amplification, where amplification is not performed if the amount is below a threshold amount.

In some embodiments, the amplification primer comprises a barcode. In general, the term "barcode" refers to a known nucleic acid sequence that allows some feature of a polynucleotide with which the barcode is associated to be identified. In some embodiments, the feature of the polynucleotide to be identified is the sample from which the polynucleotide is derived. In some embodiments, barcodes are about or at least about 3, 4, 5, 6, 7, 8, 9, 10, 11, 12, 13, 14, 15, or more nucleotides in length. In some embodiments, barcodes are shorter than 10, 9, 8, 7, 6, 5, or 4 nucleotides in length. In some embodiments, barcodes associated with some polynucleotides are of different lengths than barcodes associated with other polynucleotides. In general, barcodes are of sufficient length and comprise sequences that are sufficiently different to allow the identification of a feature of the associated polynucleotide (e.g. sample source) based on barcodes with which they are associated. In some embodiments, a barcode, and the sample source with which it is associated, can be identified accurately after the mutation, insertion, or deletion of one or more nucleotides in the barcode sequence, such as the mutation, insertion, or deletion of 1, 2, 3, 4, 5, 6, 7, 8, 9, 10, or more nucleotides. In some embodiments, each barcode in a plurality of barcodes differ from every other barcode in the plurality at at least three nucleotide positions, such as at least 3, 4, 5, 6, 7, 8, 9, 10, or more nucleotide positions. A plurality of barcodes may be represented in a pool of samples, each sample comprising polynucleotides comprising one or more barcodes that differ from the barcodes contained in the polynucleotides derived from the other samples in the pool. Samples of polynucleotides comprising one or more barcodes can be pooled based on the barcode sequences to which they are joined, such that all four of the nucleotide bases A, G, C, and T are approximately evenly represented at one or more positions along each barcode in the pool (such as at 1, 2, 3, 4, 5, 6, 7, 8, or more positions, or all positions of the barcode). In some embodiments, the methods of the invention further comprise identifying the sample from which a target polynucleotide is derived based on a barcode sequence to which the target polynucleotide is joined.

In some embodiments, separate amplification reactions are carried out for separate samples using amplification primers comprising at least one different barcode sequence for each sample, such that no barcode sequence is joined to the target polynucleotides of more than one sample in a pool of two or more samples. In some embodiments, amplified polynucleotides derived from different samples and comprising different barcodes are pooled before proceeding with subsequent manipulation of the polynucleotides (such as before amplification and/or sequencing on a solid support). Pools can comprise any fraction of the total constituent amplification reactions, including whole reaction volumes. Samples can be pooled evenly or unevenly. In some embodiments, target polynucleotides are pooled based on the barcodes to which they are joined. Pools may comprise polynucleotides derived from about, less than about, or more than about 2, 3, 4, 5, 6, 7, 8, 9, 10, 11, 12, 13, 14, 15, 16, 20, 25, 30, 40, 50, 75, 100, or more different samples. Samples can be pooled in multiples of four in order to represent all four of the nucleotide bases A, G, C, and T at one or more positions along the barcode evenly, for example 4, 8, 12, 16, 20, 24, 28, 32, 36, 40, 44, 48, 52, 56, 60, 64, 96, 128, 192, 256, 384, and so on. Non-limiting examples of barcodes include AGGTCA, CAGCAG, ACTGCT, TAACGG, GGATTA, AACCTG, GCCGTT, CGTTGA, GTAACC, CTTAAC, TGCTAA, GATCCG, CCAGGT, TTCAGC, ATGATC, and TCGGAT. In some embodiments, the barcode is positioned between sequence D and sequence C of an amplification primer, or after sequence C and sequence D in a 5' to 3' direction ("downstream"). In some embodiments, the amplification primer comprises or consists of the sequence CGAGATCTACACGCCTCCCTCGCGCCATCAGXXXXXXCACTCAGCAGCACGACGATCAC (SEQ ID NO: 21), where each "X" represents zero, one, or more nucleotides of a barcode sequence.

Non-limiting examples of amplification primers are provided in Table 1:

**Table 1:**

| | |
|---|---|
| SEQ ID NO: 1 | CGAGATCTACACGCCTCCCTCGCGCCATCAGAGGTCACACTCAGCAGCACGACGATCAC |
| SEQ ID NO: 2 | CGAGATCTACACGCCTCCCTCGCGCCATCAGCAGCAGCACTCAGCAGCACGACGATCAC |
| SEQ ID NO: 3 | CGAGATCTACACGCCTCCCTCGCGCCATCAGACTGCTCACTCAGCAGCACGACGATCAC |
| SEQ ID NO: 4 | CGAGATCTACACGCCTCCCTCGCGCCATCAGTAACGGCACTCAGCAGCACGACGATCAC |
| SEQ ID NO: 5 | CGAGATCTACACGCCTCCCTCGCGCCATCAGGGATTACACTCAGCAGCACGACGATCAC |
| SEQ ID NO: 6 | CGAGATCTACACGCCTCCCTCGCGCCATCAGAACCTGCACTCAGCAGCACGACGATCAC |
| SEQ ID NO: 7 | CGAGATCTACACGCCTCCCTCGCGCCATCAGGCCGTTCACTCAGCAGCACGACGATCAC |
| SEQ ID NO: 8 | CGAGATCTACACGCCTCCCTCGCGCCATCAGCGTTGACACTCAGCAGCACGACGATCAC |
| SEQ ID NO: 9 | CGAGATCTACACGCCTCCCTCGCGCCATCAGGTAACCCACTCAGCAGCACGACGATCAC |
| SEQID NO: 10 | CGAGATCTACACGCCTCCCTCGCGCCATCAGCTTAACCACTCAGCAGCACGACGATCAC |
| SEQ ID NO: 11 | CGAGATCTACACGCCTCCCTCGCGCCATCAGTGCTAACACTCAGCAGCACGACGATCAC |
| SEQID NO: 12 | CGAGATCTACACGCCTCCCTCGCGCCATCAGGATCCGCACTCAGCAGCACGACGATCAC |
| SEQID NO: 13 | CGAGATCTACACGCCTCCCTCGCGCCATCAGCCAGGTCACTCAGCAGCACGACGATCAC |
| SEQID NO: 14 | CGAGATCTACACGCCTCCCTCGCGCCATCAGTTCAGCCACTCAGCAGCACGACGATCAC |
| SEQID NO: 15 | CGAGATCTACACGCCTCCCTCGCGCCATCAGATGATCCACTCAGCAGCACGACGATCAC |
| SEQID NO: 16 | CGAGATCTACACGCCTCCCTCGCGCCATCAGTCGGATCACTCAGCAGCACGACGATCAC |

In some embodiments, target polynucleotides are hybridized to a plurality of oligonucleotides that are attached to a solid support. Hybridization may be before or after one or more sample processing steps, such as adapter joining and amplification. In some embodiments, target polynucleotides are hybridized to oligonucleotides on a solid support after both adapter joining and one or more amplification reactions. Oligonucleotides on the solid support may hybridize to random polynucleotide sequences, specific sequences common to multiple different target polynucleotides (e.g. one or more sequences derived from an adapter oligonucleotide, such as sequences D, D', or a portion thereof; one or more sequences derived from an amplification primer, such as sequences C, C', or a portion thereof; or combinations of these), sequences specific to different target polynucleotides (such as represented by sequence B), or combinations of these. In some embodiments, the solid support comprises a plurality of different first oligonucleotides comprising sequence A and sequence B, wherein sequence A is common among all first oligonucleotides; and further wherein sequence B is different for each different first oligonucleotide, is at the 3' end of each first oligonucleotide. In some embodiments, the plurality of first oligonucleotides comprises about, less than about, or more than about 5, 10, 25, 50, 75, 100, 125, 150, 175, 200, 300, 400, 500, 750, 1000, 2500, 5000, 7500, 10000, 20000, 50000, or more different oligonucleotides, each comprising a different sequence B. In some embodiments, sequence B or the target sequence to which it specifically hybridizes comprises a locus of interest. In some embodiments, sequence B or the target sequence to which it specifically hybridizes is within about, less than about, or more than about 1, 2, 3, 4, 5, 6, 6, 7, 8, 9, 10, 15, 20, 25, 30, 35, 40, 45, 50, 60, 70, 80, 90, 100, 200, 500 or more nucleotides of a locus of interest. The solid support may further comprise a plurality of second oligonucleotides comprising sequence A at the 3' end of each second oligonucleotide, and a plurality of third oligonucleotides comprising sequence C at the 3' end of each third oligonucleotide, as described herein.

In some embodiments, the method further comprises performing bridge amplification on the solid support. In general, bridge amplification uses repeated steps of annealing of primers to templates, primer extension, and separation of extended primers from templates. These steps can generally be performed using reagents and conditions known to those skilled in PCR (or reverse transcriptase plus PCR) techniques. Thus a nucleic acid polymerase can be used together with a supply of nucleoside triphosphate molecules (or other molecules that function as precursors of nucleotides present in DNA/RNA, such as modified nucleoside triphosphates) to extend primers in the presence of a suitable template. Excess deoxyribonucleoside triphosphates are desirably provided. Exemplary deoxyribonucleoside triphosphates are abbreviated; dTTP (deoxythymidine nucleoside triphosphate) , dATP (deoxyadenosine nucleoside triphosphate) , dCTP (deoxycytosine nucleoside triphosphate) and dGTP (deoxyguanosine nucleoside triphosphate). Exemplary ribonucleoside triphosphates are UTP, ATP, CTP and GTP. However, alternatives are possible. These may be naturally or non-naturally occurring. A buffer of the type generally used in PCR reactions may also be provided. A nucleic acid polymerase used to incorporate nucleotides during primer extension is preferably stable under the reaction conditions utilized in order that it can be used several times. Thus, where heating is used to separate a newly synthesized nucleic acid strand from its template, the nucleic acid polymerase is preferably heat stable at the temperature used. Such heat stable polymerases are obtainable from thermophilic micro-organisms, and include the DNA dependent DNA polymerase known as Taq polymerase and also thermostable derivatives thereof.

Typically, annealing of a primer to its template takes place at a temperature of 25 to 90°C. A temperature in this range will also typically be used during primer extension, and may be the same as or different from the temperature used during annealing and/or denaturation. Once sufficient time has elapsed to allow annealing and also to allow a desired degree of primer extension to occur, the temperature can be increased, if desired, to allow strand separation. At this stage the temperature will typically be increased to a temperature of 60 to 100°C. High temperatures can also be used to reduce non-specific priming problems prior to annealing, and/or to control the timing of amplification initiation, e.g. in order to synchronize amplification initiation for a number of samples. Alternatively, the strands may be separated by treatment with a solution of low salt and high pH (> 12) or by using a chaotropic salt (e.g. guanidinium hydrochloride) or by an organic solvent (e.g. formamide) .

Following strand separation (e.g. by heating), a washing step may be performed. The washing step may be omitted between initial rounds of annealing, primer extension and strand separation, such as if it is desired to maintain the same templates in the vicinity of immobilized primers. This allows templates to be used several times to initiate colony formation. The size of colonies produced by amplification on the solid support can be controlled, e.g. by controlling the number of cycles of annealing, primer extension and strand separation that occur. Other factors which affect the size of colonies can also be controlled. These include the number and arrangement on a surface of immobilized primers, the conformation of a support onto which the primers are immobilized, the length and stiffness of template and/or primer molecules, temperature, and the ionic strength and viscosity of a fluid in which the above-mentioned cycles can be performed.

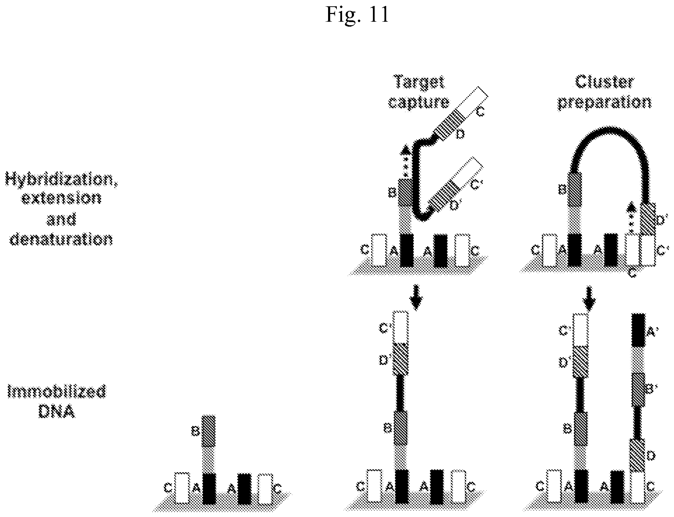

A non-limiting example of an amplification process in accordance with the methods of the disclosure is illustrated in **Fig. 11****,** and described below. First, a first oligonucleotide attached to the solid support and comprising sequence B at its 3' end hybridizes to a complementary target sequence B', such as a sequence unique to a specific target polynucleotide in a plurality of different target polynucleotides (e.g. a particular genomic DNA sequence). The target polynucleotide in **Fig. 11** comprises sequences derived from adapter oligonucleotides (e.g. sequences D and D') and from amplification primers (e.g. C and C'). Extension of the first oligonucleotide produces a first extension product attached to the solid support, the first extension product comprising, from 5' to 3', sequences A, B, C', and D', where sequence C' is complementary to sequence C and sequence D' is complementary to sequence D. The first extension product is then separated from the target polynucleotide template (e.g. by heat or chemical denaturation). Sequence C' of the first extension product then hybridizes to one of a plurality of third oligonucleotides attached to the solid support, the third oligonucleotide comprising sequence C at its 3' end. Extension of the third oligonucleotide produces a second extension product attached to the solid support, the second extension product comprising, from 5' to 3', sequences C, D, B' and A', where sequence B' is complementary to sequence B and sequence A' is complementary to sequence A. The two extension products form a double-stranded polynucleotide "bridge," with one strand at both ends attached to the solid support. The first and second extension products are then denatured, and subsequence hybridizations between the extension products and other oligonucleotides followed by extension replicate the first and second extension products. For example, each first extension product may hybridize to a further third oligonucleotide to produce additional copies of the second extension product. In addition, a second extension product may hybridize to one of a plurality of second oligonucleotides attached to the solid support, the second oligonucleotide comprising sequence A at its 3' end. Extension of the second oligonucleotide produces an extension product comprising the sequence of a first extension product. Successive rounds of extension along extension products radiates outward from an initial first extension product to produce a cluster or "colony" of first extension products and their complementary second extension products derived from a single target polynucleotide. This process may be modified to accommodate oligonucleotides comprising different sequences or sequence arrangements, different target polynucleotides or combinations of target polynucleotides, types of solid supports, and other considerations depending on a particular bridge amplification reaction. In general, this process provides for amplification on a solid support of specific target polynucleotides from sample polynucleotides comprising target polynucleotides and non-target polynucleotides. Generally, target polynucleotides are selectively amplified while non-target polynucleotides in the sample are not amplified, or are amplified to a much lower degree, such as about or less than about 10-fold, 100-fold, 500-fold, 1000-fold, 2500-fold, 5000-fold, 10000-fold, 25000-fold, 50000-fold, 100000-fold, 1000000-fold, or more lower than one or more target polynucleotides.

In some embodiments, the amount of amplified polynucleotides from a previous amplification step that is subjected to bridge amplification is about, less than about, or more than about 50ng, 100ng, 500ng, 1µg, 2µg, 3µg, 4µg, 5µg, 6µg, 7µg, 8µg, 9µg, 10µg, 11µg, 12µg, 13µg, 14µg, 15µg, 20µg, 25µg, 26µg, 27µg, 28µg, 29µg, 30µg, 40µg, 50µg, or more (e.g. a threshold amount). In some embodiments, the amount of amplified polynucleotides from a previous amplification step is determined before proceeding with bridge amplification, where bridge amplification is not performed if the amount is below a threshold amount.

In some embodiments, bridge amplification is followed by sequencing a plurality of oligonucleotides attached to the solid support. In some embodiments, sequencing comprises or consists of single-end sequencing. In some embodiments, sequencing comprises or consists of paired-end sequencing. Sequencing can be carried out using any suitable sequencing technique, wherein nucleotides are added successively to a free 3' hydroxyl group, resulting in synthesis of a polynucleotide chain in the 5' to 3' direction. The identity of the nucleotide added is preferably determined after each nucleotide addition. Sequencing techniques using sequencing by ligation, wherein not every contiguous base is sequenced, and techniques such as massively parallel signature sequencing (MPSS) where bases are removed from, rather than added to the strands on the surface are also within the scope of the invention, as are techniques using detection of pyrophosphate release (pyrosequencing). Such pyrosequencing based techniques are particularly applicable to sequencing arrays of beads where the beads have been amplified in an emulsion such that a single template from the library molecule is amplified on each bead.

One particular sequencing method which can be used in the methods of the invention relies on the use of modified nucleotides that can act as reversible chain terminators. Such reversible chain terminators comprise removable 3' blocking groups, for example as described in WO04018497 and US7057026. Once such a modified nucleotide has been incorporated into the growing polynucleotide chain complementary to the region of the template being sequenced there is no free 3'-OH group available to direct further sequence extension and therefore the polymerase cannot add further nucleotides. Once the identity of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached thereto a different label, known to correspond to the particular base, to facilitate discrimination between the bases added at each incorporation step. Non-limiting examples of suitable labels are described in WO/2007/135368. Alternatively, a separate reaction may be carried out containing each of the modified nucleotides added individually.

The modified nucleotides may carry a label to facilitate their detection. In a particular embodiment, the label is a fluorescent label. Each nucleotide type may carry a different fluorescent label. However, the detectable label need not be a fluorescent label. Any label can be used which allows the detection of the incorporation of the nucleotide into the DNA sequence. One method for detecting fluorescently labeled nucleotides comprises using laser light of a wavelength specific for the labeled nucleotides, or the use of other suitable sources of illumination. Fluorescence from the label on an incorporated nucleotide may be detected by a CCD camera or other suitable detection means. Suitable detection means are described in WO/2007/123744.

In some embodiments, a first sequencing reaction proceeds from a 3' end created by cleavage at a cleavage site contained in an oligonucleotide attached to the solid support, which oligonucleotide was extended during bridge amplification. In some embodiments, the cleaved strand is separated from its complementary strand before sequencing by extension of the attached oligonucleotide. In some embodiments, the attached oligonucleotide having the newly freed 3' end created by cleavage is extended using a polymerase having strand displacement activity, such that the cleaved strand is displaced as the new strand is extended. In some embodiments, extension of the attached oligonucleotide proceeds along the full length of the template extension product from the amplification reaction, which in some embodiments includes extension beyond a last identified nucleotide. In some embodiments, the template extension product is then cleaved at a cleavage site contained in an oligonucleotide attached to the solid support, and the oligonucleotide extended during the sequencing reaction is linearized, for produce a freed first sequencing extension product. The 5' end of the first sequencing product may then serve as a template for a second sequencing reaction, which can proceed by extension of a sequencing primer (such as a sequencing primer described herein) or by extension from the 3' end created by cleavage at the cleavage site. In some embodiments, the average or median number of nucleotides identified along a template polynucleotide being sequenced is about, less than about, or more than about 10, 15, 20, 25, 30, 35, 40, 45, 50, 60, 70, 80, 90, 100, 150, 200, 300, 400, 500, or more.

In some embodiments, sequencing comprises treating bridge amplification products to remove substantially all or remove or displace at least a portion of one of the immobilized strands in the "bridge" structure in order to generate a template that is at least partially single-stranded. The portion of the template which is single-stranded will thus be available for hybridization with a sequencing primer. The process of removing all or a portion of one immobilized strand in a bridged double-stranded nucleic acid structure may be referred to herein as "linearization," and is described in further detail in WO07010251.

Bridged template structures may be linearized by cleavage of one or both strands with a restriction endonuclease or by cleavage of one strand with a nicking endonuclease. Other methods of cleavage can be used as an alternative to restriction enzymes or nicking enzymes, including but not limited to chemical cleavage (e.g. cleavage of a diol linkage with periodate), cleavage of abasic sites by cleavage with endonuclease (for example "USER," as supplied by NEB, part number M5505S), by exposure to heat or alkali, cleavage of ribonucleotides incorporated into amplification products otherwise comprised of deoxyribonucleotides, photochemical cleavage or cleavage of a peptide linker. In some embodiments, a linearization step may be avoided, such as when the solid-phase amplification reaction is performed with only one amplification oligonucleotide covalently immobilized and another amplification oligonucleotide free in solution. Following the cleavage step, regardless of the method used for cleavage, the product of the cleavage reaction may be subjected to denaturing conditions in order to remove the portion(s) of the cleaved strand(s) that are not attached to the solid support. Suitable denaturing conditions, for example sodium hydroxide solution, formamide solution, or heat, are known in the art, such as described in standard molecular biology protocols (Sambrook et al., 2001, Molecular Cloning, A Laboratory Manual, 3rd Ed, Cold Spring Harbor Laboratory Press, Cold Spring Harbor Laboratory Press, NY; Current Protocols, eds Ausubel et al.). Denaturation results in the production of a sequencing template which is partially or substantially single-stranded. A sequencing reaction may then be initiated by hybridization of a sequencing primer to the single-stranded portion of the template. Thus, the disclosure encompasses methods wherein the nucleic acid sequencing reaction comprises hybridizing a sequencing primer to a single-stranded region of a linearized amplification product, sequentially incorporating one or more nucleotides into a polynucleotide strand complementary to the region of amplified template strand to be sequenced, identifying the base present in one or more of the incorporated nucleotide(s) and thereby determining the sequence of a region of the template strand.

In some embodiments, the sequencing primer comprises a sequence complementary to one or more sequences derived from an adapter oligonucleotide, an amplification primer, an oligonucleotide attached to the solid support, or a combination of these. In some embodiments, the sequencing primer comprises sequence D, or a portion thereof. In some embodiments, a sequencing primer comprises sequence C, or a portion thereof. A sequencing primer can be of any suitable length, such as about, less than about, or more than about 5, 10, 15, 20, 25, 30, 35, 40, 45, 50, 55, 60, 65, 70, 75, 80, 90, 100, or more nucleotides, any portion or all of which may be complementary to the corresponding target sequence to which the primer hybridizes (e.g. about, less than about, or more than about 5, 10, 15, 20, 25, 30, 35, 40, 45, 50, or more nucleotides). In some embodiments, a sequencing primer comprises the sequence CACTCAGCAGCACGACGATCACAGATGTGTATAAGAGACAG (SEQ ID NO: 20).

In general, extension of a sequencing primer produces a sequencing extension product. The number of nucleotides added to the sequencing extension product that are identified in the sequencing process may depend on a number of factors, including template sequence, reaction conditions, reagents used, and other factors. In some embodiments, the average or median number of nucleotides identified along a growing sequencing primer is about, less than about, or more than about 10, 15, 20, 25, 30, 35, 40, 45, 50, 60, 70, 80, 90, 100, 150, 200, 300, 400, 500, or more. In some embodiments, a sequencing primer is extended along the full length of the template primer extension product from the amplification reaction, which in some embodiments includes extension beyond a last identified nucleotide.

In some embodiments, the sequencing extension product is subjected to denaturing conditions in order to remove the sequencing extension product from the attached template strand to which it is hybridized, in order to make the template partially or completely single-stranded and available for hybridization with a second sequencing primer. The second sequencing primer may be the same as or different from the first sequencing primer. In some embodiments, the second sequencing primer hybridizes to a sequence located closer to the 5' end of the target nucleic acid than the sequence to which the first sequencing primer hybridizes. In some embodiments, the second sequencing primer hybridizes to a sequence located closer to the 3' end of the target nucleic acid than the sequence to which the first sequencing primer hybridizes. In some embodiments, only one of the first and second sequencing primers is extended along a barcode sequence, thereby identifying the nucleotides in the barcode sequence. In some embodiments, one sequencing primer (e.g. the first sequencing primer) hybridizes to a sequence located 5' from the barcode (such that extension of this sequencing primer does not generate sequence complementary to the barcode), and another sequencing primer (e.g. the second sequencing primer) hybridizes to a sequence located 3' from the barcode (such that extension of this sequencing primer generates sequence complementary to the barcode). In some embodiments, the second sequencing primer comprises SEQ ID NO: 19.

The dislcosure is not intended to be limited to use of the sequencing methods outlined above, as a variety of sequencing methodologies that rely on successive incorporation of nucleotides into a polynucleotide chain can be used. Suitable techniques include, for example, those described in US6306597, US20090233802, US20120053074, and US20110223601. In the cases where strand resynthesis is employed, both strands must be immobilized to the surface in a way that allows subsequent release of a portion of the immobilized strand. This can be achieved through a number of mechanisms as described in WO07010251. For example, one primer can contain a uracil nucleotide, which means that the strand can be cleaved at the uracil base using the enzyme uracil DNA glycosylase (UDG) which removes the nucleotide base, and endonuclease VIII that excises the abasic nucleotide. This enzyme combination is available as USER^{™} from New England Biolabs (NEB part number M5505). The second primer may comprise an 8-oxoguanine nucleotide, which is then cleavable by the enzyme FPG (NEB part number M0240). This design of primers provides complete control of which primer is cleaved at which point in the process, and also where in the cluster the cleavage occurs. The primers may also be chemically modified, for example with a disulfide or diol modification that allows chemical cleavage at specific locations.

In some embodiments, sequencing data are generated for about, less than about, or more than about 5, 10, 25, 50, 100, 150, 200, 250, 300, 400, 500, 750, 1000, 2500, 5000, 7500, 10000, 20000, 50000, or more different target polynucleotides from a sample in a single reaction container (e.g. a channel in a flow cell). In some embodiments, sequencing data are generated for a plurality of samples in parallel, such as about, less than about, or more than about 2, 3, 4, 5, 6, 7, 8, 9, 10, 11, 12, 13, 14, 15, 16, 17, 18, 19, 20, 24, 48, 96, 192, 384, 768, 1000, or more samples. In some embodiments, sequencing data are generated for a plurality of samples in a single reaction container (e.g. a channel in a flow cell), such as about, less than about, or more than about 2, 3, 4, 5, 6, 7, 8, 9, 10, 11, 12, 13, 14, 15, 16, 17, 18, 19, 20, 24, 48, 96, 192, 384, 768, 1000, or more samples, and sequencing data are subsequently grouped according to the sample from which the sequenced polynucleotides originated. In a single reaction, sequencing data may be generated for about or at least about 10⁶, 10⁷,10⁸, 2×10⁸, 3×10⁸, 4×10⁸, 5×10⁸, 10⁹, 10¹⁰, or more target polynucleotides or clusters from a bridge amplification reaction, which may comprise sequencing data for about, less than about, or more than about 10⁴, 10⁵, 10⁶, 2×10⁶, 3×10⁶, 4x10⁶, 5×10⁶, 10⁷, 10⁸, or more target polynucleotides or clusters for each sample in the reaction. In some embodiments, the presence, absence, or genotype of about, less than about, or more than about 5, 10, 25, 50, 75, 100, 125, 150, 175, 200, 300, 400, 500, 750, 1000, 2500, 5000, 7500, 10000, 20000, 50000, or more causal genetic variants is determined for a sample based on the sequencing data. The presence, absence, or genotype of one or more causal genetic variants may be determined with an accuracy of about or more than about 80%, 85%, 90%, 95%, 97.5%, 99%, 99.5%, 99.9% or higher.

In some embodiments, one or more, or all, of the steps in a method of the invention are automated, such as by use of one or more automated devices. In general, automated devices are devices that are able to operate without human direction-an automated system can perform a function during a period of time after a human has finished taking any action to promote the function, e.g. by entering instructions into a computer, after which the automated device performs one or more steps without further human operation. Software and programs, including code that implements embodiments of the present invention, may be stored on some type of data storage media, such as a CD-ROM, DVD-ROM, tape, flash drive, or diskette, or other appropriate computer readable medium. Various embodiments of can also be implemented exclusively in hardware, or in a combination of software and hardware. For example, in one embodiment, rather than a conventional personal computer, a Programmable Logic Controller (PLC) is used. As known to those skilled in the art, PLCs are frequently used in a variety of process control applications where the expense of a general purpose computer is unnecessary. PLCs may be configured in a known manner to execute one or a variety of control programs, and are capable of receiving inputs from a user or another device and/or providing outputs to a user or another device, in a manner similar to that of a personal computer. Accordingly, although embodiments are described in terms of a general purpose computer, it should be appreciated that the use of a general purpose computer is exemplary only, as other configurations may be used.

In some embodiments, automation may comprise the use of one or more liquid handlers and associated software. Several commercially available liquid handling systems can be utilized to run the automation of these processes (see for example liquid handlers from Perkin-Elmer, Beckman Coulter, Caliper Life Sciences, Tecan, Eppendorf, Apricot Design, Velocity 11 as examples). In some embodiments, automated steps include one or more of fragmentation, end-repair, A-tailing (addition of adenine overhang), adapter joining, PCR amplification, sample quantification (e.g. amount and/or purity of DNA), and sequencing. In some embodiments, hybridization of amplified polynucleotides to oligonucleotides attached to a solid surface, extension along the amplified polynucleotides as templates, and/or bridge amplification is automated (e.g. by use of an Illumina cBot). Non-limiting examples of devices for conducting bridge amplification are described in WO2008002502. In some embodiments, sequencing is automated. A variety of automated sequencing machines are commercially available, and include sequencers manufactured by Life Technologies (SOLiD platform, and pH-based detection), Roche (454 platform), Illumina (e.g. flow cell based systems, such as Genome Analyzer, HiSeq, or MiSeq systems). Transfer between 2, 3, 4, 5, or more automated devices (e.g. between one or more of a liquid handler, bridge a amplification device, and a sequencing device) may be manual or automated. In some embodiments, one or more steps in a method of the invention (e.g. all steps or all automated steps) are completed in about or less than about 72, 48, 24, 20, 18, 16, 14, 12, 10, 9, 8, 7, 6, 5, 4, 3, 2, 1, or fewer hours. In some embodiments, the time from sample receipt, DNA extraction, fragmentation, adapter joining, amplification, or bridge amplification to production of sequencing data is about or less than about 72, 48, 24, 20, 18, 16, 14, 12, 10, 9, 8, 7, 6, 5, 4, 3, 2, 1, or fewer hours.

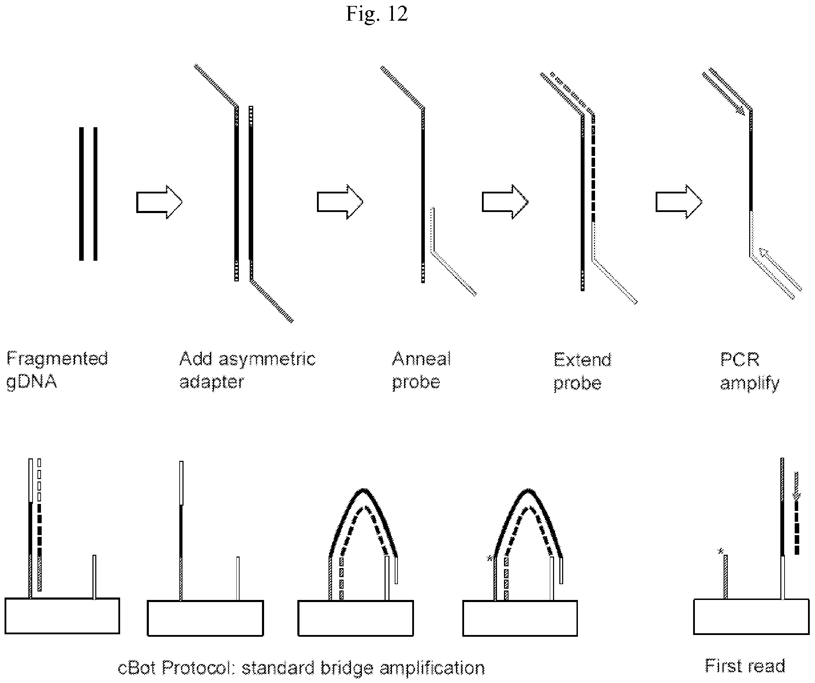

An example embodiment of a process comprising adding an adapter, extension of a first primer, amplification with a pair of primers, bridge amplification of target polynucleotides, and sequencing is illustrated in **Fig. 12****.** Additional methods and compositions for amplifying and sequencing target polynucleotides are described in US 20140024536 and WO2014015084A2.

### V. Probe Design

The systems and methods of this disclosure generally provide for use of set of at least two probes or hybridization sequences, herein known as a "probe set". Probes are designed to contain sequences that selectively anneal or hybridize to at least two complementary regions of a locus of a polynucleotide sequences. Generally, probes of a probe set are designed to anneal to the same, or identical, polynucleotide strand. In some cases, more than two probes may be used. In this disclosure, when two probes are used, probes may be described as a "first probe" and "second probe". Generally, probes may exist as separate, non-contiguous oligonucleotides, before hybridization to a sample polynucleotide. In other cases, one or more probes may be connected by a linker sequence, such as found in a molecular inversion probe (MIP) or padlock probe before hybridization to a sample polynucleotide. Generally, probes comprise a single stranded polynucleotide molecule. In some cases, probes may comprise DNA. Generally probes are artificial sequences, or sequences comprising nucleotide species not originally present in the sample polynucleotides.

In the case of two hybridization sequences of a probe set, the first probe and second probe are generally designed to hybridize or anneal to target elements in a polynucleotide sequence corresponding to a locus or region for testing. In some cases the target elements may be sequences such as found in gDNA of the locus sequence. In some cases the target elements may be sequences as found in mRNA transcripts or cDNA sequences. In some cases, target elements may be adapter sequences, which may be attached (i.e. ligated, conjugated etc...) to the ends of polynucleotides and may be not be natively found in the sequence of the polynucleotide. Adapter sequences may be attached to polynucleotides or polynucleotide fragments in steps prior to hybridization using any suitable methods known in the art as described herein. In one example, a first probe or second probe may be designed to anneal or hybridize to one or more adapter sequences attached to a polynucleotide corresponding to a locus.

In some cases, one or more probes may be about 10%-30%, 30%-60%, 60%-90%, or 90%-99.99% complementary to a sequence in a locus. In some cases, one or more probes may be at least about 10%, 20%, 30%, 40%, 50%, 60%, 70%, 80%, 90%, 95%, 99%, 99.9%, 99.99% complementary to a sequence in a locus. In some cases, probes may be at most about 10%, 20%, 30%, 40%, 50%, 60%, 70%, 80%, 90%, 95%, 99%, 99.9%, 99.99% complementary to a sequence in a locus.

In some cases, one or more probes may be designed to selectively detect a single nucleotide polymorphism (SNP). In some cases, two different probe sets (each probe set assigned with a different barcode, as described herein), may be used for an identical region in the same locus. The two probe sets may be designed to detect a putative SNP in locus, such that one probe set, comprising one variant SNP sequence is able to hybridize to the locus, while the other probe set, containing an alternative variant SNP sequence is not able to hybridize to the locus. Detection of the polymorphism may be detected through the successful hybridization of a particular probe in a probe set with the SNP and the enumeration of the barcode sequence assigned to the respective probe set. In some cases, one or more variable bases for the detection of SNPs may be located at the 3' end of either the first probe, second probe or both probes. In some cases, one or more variable bases for the detection of SNPs may be located near the intended ligation point between the first probe and second probe.

Further, the systems and methods of this disclosure provide for any suitable algorithms that may allow for optimal probe selection throughout an entire genome. Algorithms may aid in probe design or probe selection, such as providing recommendations for optimal sequences within a locus, providing recommendations for length of the probe sequence, as well as providing general optimal parameters for hybridization (i.e. temperature, salt concentration etc...). In some cases, algorithms may be used to select specific loci in a chromosome or one or more loci of interest in a plurality of chromosomes.

In some cases, the first and second probe may be about 5-50, 50-100, 100-200, 200-300, 300-400, or 400-500 base pairs in length. In some cases, the first and second probe may be at least about 5, 10, 15, 20, 25, 30, 35, 40, 45, 50, 55, 60, 65, 70, 75, 80, 85, 90, 95, 100, 125, 150, 175, 200, 300, 400 or 500 base pairs in length. In some cases, the first and second probe may be at most about 5, 10, 15, 20, 25, 30, 35, 40, 45, 50, 55, 60, 65, 70, 75, 80, 85, 90, 95, 100, 125, 150, 175, 200, 300, 400 or 500 base pairs in length.

Further, the first and second probe may be designed to bind adjacent sites on an identical strand of a polynucleotide sequence. In some cases a gap, or a region of single stranded sample sequence to which a probe has not annealed or hybridized, exists between the first and second probes. In some cases no gap exists between the binding sites of the first and second probe, such that the first and second probe may be directly ligated as described herein. In some cases the gap may be about 1-25 bp, 25-50 bp, 50-100 bp, 100-500 bp, 500-1 Kb, 1Kb-2Kb, 2Kb-3Kb, 3Kb-4Kb, or 4Kb-5Kb in length. In some cases the gap may be at least about 1 bp, 2 bp, 3 bp, 4 bp, 5 bp, 10 bp, 15 bp, 20 bp, 25 bp, 30 bp, 35 bp, 40 bp, 45 bp, 50 bp, 75 bp, 100, bp 125 bp, 150 bp, 175 bp, 200 bp, 300 bp, 400 bp, 500 bp, 600 bp, 700 bp, 800 bp, 900 bp, 1 Kb, 2 Kb, 3 Kb, 4 Kb, 5 Kb, or 10 Kb in length. In some cases the gap may be at most about 1 bp, 2 bp, 3 bp, 4 bp, 5 bp, 10 bp, 15 bp, 20 bp, 25 bp, 30 bp, 35 bp, 40 bp, 45 bp, 50 bp, 75 bp, 100, bp 125 bp, 150 bp, 175 bp, 200 bp, 300 bp, 400 bp, 500 bp, 600 bp, 700 bp, 800 bp, 900 bp, 1 kb, 2 kb, 3 kb, 4 kb, 5 kb, or 10 kb in length.

Generally, the number of probe sets used may be determined by the number loci to be tested in a sample. In some cases, about 2-100, 100-500, 500-1000, 1000-2000, 2000-3000, 3000-4000, 4000-5000, 5000-6000, 6000-7000, 7000-8000, 8000-9000, or 9000-10000 probe sets may be used. In some cases, at least about 2, 3, 4, 5, 6, 7, 8, 9, 10, 20, 30, 40, 50, 60, 70, 80, 90, 100, 200, 300, 400, 500, 600, 700, 800, 900, 1000, 1100, 1200, 1300, 1400, 1500, 1600, 1700, 1800, 1900, 2000, 3000, 4000, 5000, 6000, 7000, 8000, 9000, or 10000 probe sets may be used. In some cases, at most about 2, 3, 4, 5, 6, 7, 8, 9, 10, 20, 30, 40, 50, 60, 70, 80, 90, 100, 200, 300, 400, 500, 600, 700, 800, 900, 1000, 1100, 1200, 1300, 1400, 1500, 1600, 1700, 1800, 1900, 2000, 3000, 4000, 5000, 6000, 7000, 8000, 9000, or 10000 probe sets may be used.

Further, in some cases, about 2-100, 100-500, 500-1000, 1000-2000, 2000-3000, 3000-4000, 4000-5000, 5000-6000, 6000-7000, 7000-8000, 8000-9000, or 9000-10000 probes may be used to test a single locus. In some cases, at least about 1, 2, 3, 4, 5, 6, 7, 8, 9, 10, 20, 30, 40, 50, 60, 70, 80, 90, 100, 200, 300, 400, 500, 600, 700, 800, 900, 1000, 1100, 1200, 1300, 1400, 1500, 1600, 1700, 1800, 1900, 2000, 3000, 4000, 5000, 6000, 7000, 8000, 9000, or 10000 probes may be used to test a single locus. In some cases, at most about 1, 2, 3, 4, 5, 6, 7, 8, 9, 10, 20, 30, 40, 50, 60, 70, 80, 90, 100, 200, 300, 400, 500, 600, 700, 800, 900, 1000, 1100, 1200, 1300, 1400, 1500, 1600, 1700, 1800, 1900, 2000, 3000, 4000, 5000, 6000, 7000, 8000, 9000, or 10000 probes may be used to test a single locus.

### VI. Loci of Interest and Genetic Alterations

The systems and methods of this disclosure provide for testing of one or more loci in a genome. Generally, a locus may comprise any sequence of interest in sample. In some cases, a loci may be described as a "loci of interest," generally referring to a locus with a putative genetic alteration, as further described herein. In some aspects of this disclosure, at least one locus of interest and a locus outside of the locus of interest are tested. In some cases, a locus or locus of interest may be any suitable sequence in a sample. A locus or locus of interest may include, but are not limited to, a chromosome, gene, exon, intron, intron-exon boundary, promoter, terminator, highly repetitive sequence, LTR, UTR, satellite sequences, centromere repeats, telomeres, non-coding sequences, coding sequences, regulators, plasmids, transcription factor binding sites, ribosomal binding sites, 5' cap, poly d(T) sequence epigenetic sequences, mobile elements, transposons or combination thereof. In some cases, a loci may be in any polynucleotide sequences in a cell. In some cases, such as humans, a locus may comprise the full sequence or a partial sequence of mitochondrial gDNA, chromosome 1, chromosome 2, chromosome 3, chromosome 4, chromosome 5, chromosome, 6, chromosome, 7, chromosome 8, chromosome 9, chromosome, 10, chromosome 11, chromosome 12, chromosome 13, chromosome 14, chromosome 15, chromosome 16, chromosome 17, chromosome 18, chromosome 19, chromosome 20, chromosome 21, chromosome 22, X chromosome or Y chromosome.

In some cases, about 2-100, 100-500, 500-1000, 1000-2000, 2000-3000, 3000-4000, 4000-5000, 5000-6000, 6000-7000, 7000-8000, 8000-9000, or 9000-10000 loci may be tested in a sample. In some cases, at least about 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000 loci may be tested in a sample. In some cases, at most about 1, 2, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, or 10000 loci may be tested in a sample.

In some instances, loci may be selectively chosen based on relative binding affinity of certain probes, or based on association with certain diseases as further described herein. For example, multiple loci, known to be associated with a disease such as autism, may be selected and tested. In some cases testing loci may be performed simultaneously or sequentially. In some cases, multiple loci for multiple diseases may be chosen. Testing of one or more loci may be performed simultaneously or sequentially. For example loci associated with autism and loci associated with Trisomy 21 may be simultaneously tested. In another example, one or more loci may be chosen for specific chromosomes, such as 21, 18 and 13 which may comprise copy number variations. In other cases loci may be chosen to represent sequences throughout the genome and may not be associated with one or known diseases.

Further, in some cases, within a locus, probes may be chosen to target polymorphic sequences. In some cases, polymorphic sequences may contain SNPs. In other cases, polymorphic sequences may not contain SNPs. In some cases, within a locus, probes maybe chosen to target non-polymorphic sequences.

The systems and methods of this disclosure provide for the detection of various types of genetic alterations, which may or may not be found in a locus or locus of interest. "Genetic alterations," "genetic abnormalities" and "chromosomal abnormalities" are used interchangeably herein. In some cases, genetic alterations may be CNVs. In other cases, genetic alterations may be loss of heterozygosity (LOH). Generally, genetic alterations may include, but are not limited to rearrangements, subtelomeric rearrangement, aneupoloidy, partial aneulpoidy, polypoloidy, chromosomal instability, mutations, rare mutations, copy number variations, transversions, translocations, inversion, indels, deletions, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, abnormal changes in nucleic acid methylation, viral insertions, parasitic DNA insertions, alterations in tandem repeats, infection and cancer.

### VII. Barcodes and Additional Sequences

In some instances, additional sequences are found in combination with the probe or hybridization sequences in a probe set. Generally, barcode sequences, adapter sequences, universal primer sequences and other linker sequences may be linked to the first and/or second probe.

In cases in which the first and second probes may be linked, such as provided by a MIP or padlock probe, barcode sequences, adapter sequences, universal primer sequences and other linker sequences may be found in the contiguous looping region linking the two probes. In other cases, in which the probes remain linear, additional sequences may be found in non-complementary regions of the 5' region of either probe.

### A. Molecular Barcoding Probe Sets

The systems and methods of this disclosure may also enable the probe sequences to be assigned an identifier. Assignment of an identifier may allow tagging, tracking, or barcoding of probe sequences in order to permit subsequent identification of particular probe sequences that bind to polynucleotide sample strands. Assignment of an identifier may also provide a means for quantification or enumeration of the identifier and subsequent enumeration of loci in the sample.

In some cases, an identifier may be an oligonucleotide barcode sequence. In some examples, the identity of a barcode sequence associated with a probe set may be known before use of the probe set for hybridization and downstream analyses. In some cases, the barcode sequence may be representative of the probe sequence, such that enumeration of the copy number of barcode sequences in downstream steps, may be representative of the copy number of bound probes in a sample. Further, the copy number of bound probes may be representative of the copy number of sequences present in a locus or locus of interest. In some cases, the copy number of barcodes may be directly proportional to the copy number of probes and subsequently, the copy number of sequences in a locus of interest. In some cases, the copy numbers of barcodes may be equal to the copy number of probes or copy number of sequences in a locus of interest. Thus, enumeration of barcodes, probes, or combination thereof may be used to determine copy number of a particular sequence in a locus.

The identity and enumeration of barcode sequences may be performed with various techniques as further described herein. For example, sequencing may be used to enumerate barcodes, as also further described herein.

Further, in some cases, a fully unique barcode sequence may be assigned to individual probe sets. In this case, a unique barcode sequence may be attached to one or more probes in a probe set. In some cases, one probe set may be designed to test one locus. Detection and enumeration of the unique barcode sequence may provide the identity and abundance of the corresponding locus.

In some cases, unique barcodes may be formed from the combination of various sequences. In some cases, non-unique barcodes may be linked with additional sequences such as probe sequences, portions of probe sequences or additional sequences linked to the probe to form a unique barcode sequence. For example, the formation of a unique sequence may be formed at the beginning (start) and end (stop) portions of the probe sequences when used, alone or in combination, with a non-unique bar code sequence. The combination of sequences (i.e. probe sequence and non-unique barcode sequence), may provide unique identifying sequences. For example, in some cases a barcode may be designed with a general structure, 5'XXXXYYYY, wherein X is a variable length region complementary to one sequence selected from sequences including but not limited to first and/or second probe/hybridization sequences, adapter sequences, universal priming sequences, or linker sequences. Y may be selected from a non-unique barcode sequence of variable length. In some cases, Y sequences may be common to all probe sets in a sample. In other cases, Y sequences may be unique one locus, or a plurality of loci, such as a whole chromosome, or loci associated with a particular disease or genotype. In some cases, the length, or number of nucleotides defined as either X or Y may be about 1-20, 20-50, 50-75, 75-100, 100-150, 150-200, 200- 300, 300- 400 or 400-500 nucleotides. In some cases, the length, or number of nucleotides defined as either X or Y may be at least about 1, 2, 3, 4, 5, 6, 7, 8, 9, 10, 15, 20, 25, 30, 35, 40, 45, 50, 75, 100, 125, 150, 175, 200, 300, 400 or 500 nucleotides. In some cases, the length, or number of nucleotides defined as either X or Y may be at most about 1, 2, 3, 4, 5, 6, 7, 8, 9, 10, 15, 20, 25, 30, 35, 40, 45, 50, 75, 100, 125, 150, 175, 200, 300, 400 or 500 nucleotides.

In alternative configurations, barcodes may be formed through the combination of at least about 1, 2, 3, 4, 5, 6, 7, 8, 9 or 10 sequences. Barcodes may be formed the combination of at most about 1, 2, 3, 4, 5, 6, 7, 8, 9 or 10 non-unique sequences.

In alternative configurations of the systems and methods of this disclosure, non-unique barcode sequences may also be assigned to one or more probe sets. For example, identical barcodes may be assigned to probes complementary for the same locus. In some cases, the locus may be a chromosomal region. In other cases a locus may be an entire chromosome, wherein probes sets designed to hybridize to multiple regions of the same chromosome may be assigned identical barcode sequences. In this example, barcode sequences may be unique for a particular chromosome, but similar for loci on the same chromosome. For example, one particular barcode ("A") may be assigned to probes or probe sets designed for a locus of interest, such as chromosome 21, while another barcode of differing sequence ("B") may be assigned to probes designed for another locus of interest, chromosome 18. Further, a third barcode ("C") may be assigned to a locus outside of either chromosome 21 or chromosome 2 to provide a reference. Enumeration of barcodes A, B and C, and comparison of enumerated reads of A to C and B to C may be used to detect genetic alterations, such as copy number variation of either chromosome 21 or chromosome 18 as a whole.

Additionally, common barcode sequences may be assigned to one or more loci associated with a disease or disease state. For example, one barcode sequence ("X") may be assigned to one or more probes complementary to one or more loci associated with cystic fibrosis, while another barcode ("Y") is assigned to probes designed for loci associated with downs syndrome. A third barcode ("Z") may be assigned to probes designed to detect a locus not associated with either disease. Enumeration of barcodes X, Y and Z, and comparison of enumerated reads of X to Z and Y to Z may be used to detect genetic alterations of either disease in a single assay. In some cases, loci associated with a particular disease may be found across multiple chromosomes. In this manner, one or more loci, associated with one or more diseases, may be tested simultaneously. Alternatively, one or more loci, associated with one or more diseases, may be tested sequentially.

In some cases, the barcode sequences maybe about 1-10,10-20,20-50,50-100,100-500, or 500-1000 base pairs. In some cases, the barcode sequences may be a variety of lengths such that each barcode is at least about 1, 2, 3, 4, 5, 6, 7, 8, 9, 10, 20, 50, 100, 500, or 1000 base pairs. In other cases, the barcodes may comprise less than 1, 2, 3, 4, 5, 6, 7, 8, 9, 10, 20, 50, 100, 500, or 1000 base pairs.

### B. Assignment of Barcodes to Probe Sets

Often, as described herein, an identifier assigned to a probe set is an oligonucleotide barcode sequence that is contiguous with the first or second probe in a probe set. In some cases, however, different identifiers may be used. An identifier, as with barcode sequences, may be unique or non-unique. For example, in some cases, the unique identifier may be a hybridization probe. In one example, a hybridization probe may comprise an oligonucleotide sequence and an additional component such as fluorescent element (i.e. nanoparticle, nanoprobe, quantum dot, etc). In some cases, one or more fluorescent elements may be described as barcodes as well. For example, fluorescent elements of varying wavelengths or colors may be arrayed in unique or non unique patterns or sequences. In other cases, the identifier is a dye, in which case the attachment may comprise intercalation of the dye into the analyte molecule (such as intercalation into DNA or RNA) or binding to a probe labeled with the dye. In still other cases, the identifier may be a nucleic acid oligonucleotide, in which case the attachment to the polynucleotide sequences may comprise a ligation reaction between the oligonucleotide and the sequences or incorporation through PCR. In other cases, the reaction may comprise addition of a metal isotope, in which either the first or second probe is labeled with the isotope.

In cases in which identifier comprises barcode sequences, the systems and methods of this disclosure provide for any suitable reagents necessary for the assignment of barcodes to probes. In some cases, ligation reactions may be utilized, in which reagents including, but not limited to, ligase enzyme, probes, buffer, adapter oligonucleotides, and a plurality of identifier DNA barcodes may be used to generated probe sets. In the case of enrichment methods, reagents including but not limited to a plurality of PCR primers, probes, oligonucleotides containing barcode sequences, DNA polymerase, DNTPs, and buffer and the like may be used in preparation of the probe sets.

### VIII. Hybridization and Ligation

Generally, any suitable conditions may be used to hybridize probe sets to a sample polynucleotide. In some instances, the first probe and second probes anneal sequentially. In other instances, the first and second probes anneal simultaneously. In some cases, probes may be added to a hybridization reaction sequentially. In some cases, probes may be added to a hybridization reaction simultaneously, such as with MIP or padlock probes, in which probes are linked. The binding of a first and second probe generally provide for high selectivity of binding, especially when used in a MIP or padlock configuration. Generally, use of this configuration in this disclosure provides for reduction of non-specific binding of probes. Varying hybridization conditions, such as salt concentrations, temperature, polynucleotide concentrations, pH etc... may also be used to reduce non-specific binding of probes to sample DNA.

Generally, after hybridization, the first and second probes are ligated together to form a ligation product. Generally, ligation products comprise artificial sequences, or sequences comprising nucleotide species not originally present in the sample polynucleotides.

In some instances, the probes may be designed to hybridize to sites directly adjacent to one another, such that no gap exists between them when hybridized to a sample polynucleotide. In this case, addition of ligase is sufficient to form a contiguous ligation product between the first and second probe. In cases involving two non-contiguous probes, the ligation product is a contiguous linear polynucleotide. In cases involving MIP or padlock probes, ligation of the first and second probe create a fully circular contiguous sequences, as known in the art.

In other instances, the first and second probes may not be directly adjacent, wherein a gap exists between the probes, as described herein. In some cases, ligation may be preceded or accompanied by additional steps, such as a primer extension step using a polymerase, and/or use of bridging oligonucleotides as described herein.

### A. Polymerase Extension Step

In some configurations, the first and second probe may be positioned such that an additional step is necessary. In some instances, where the first and second probes are not directly adjacent to one another, an extension step may be required. In some cases, a polymerase and dNTPs may be used to polymerize complementary sequence to fill the gap between the two probes, wherein sequence from the 3' end of the first probe is extended across the gap. In some instances, this configuration may be useful, wherein optimal annealing sites for the first and second probes may not be directly adjacent to one another. In some cases, an extension step may also be useful, wherein a binding partner may be incorporated into the polynucleotide product in the polymerized complementary sequence. For example, dNTPs conjugated with a binding partner such as biotin, may be used, such that biotinylated dNTPs may be incorporated into the ligation product. This may be useful for affinity purification of the product in subsequent steps with additional binding partners, such as streptavidin. Affinity purification may be useful in various steps such as separation of the contiguous ligation product from sample DNA or gDNA or for the enrichment of certain ligation products as described herein.

### B. Bridging Oligos

In some configurations, a bridging oligo may be used in combination with the first and second probes. Generally, a bridging oligo may comprise a polynucleotide sequence capable of binding to a sequence found between regions annealing to either the first or second probe in the locus or locus of interest. In some cases 1 bridging oligo may be used. In some cases at least about 1, 2, 3, 4, 5, 6, 7, 8, 9 or 10 bridging oligos are used. In some cases at most about 1, 2, 3, 4, 5, 6, 7, 8, 9 or 10 bridging oligos are used. One or more bridging oligos may be added either sequentially or simultaneously with one another and/or the addition of either the first or second probe.

The bridging oligo may be complimentary to any sequence found between regions annealing to either the first or second probe in the locus of interest. In some cases the bridging oligo may anneal to a region containing an A/T or G/C SNP. In other cases, a bridging oligo may be 100% complementary to sequences in the locus of interest. In some cases, a bridging oligo may be at least about 10%, 20%, 30%, 40%, 50%, 60%, 70%, 80%, 90%, 95%, 99%, or 99.99% complementary to a sequence found between regions annealing to either the first or second probe. In some cases, a bridging oligo may be at most about 10%, 20%, 30%, 40%, 50%, 60%, 70%, 80%, 90%, 95%, 99%, or 99.99% complementary to a sequence found between regions annealing to either the first or second probe.

In some cases, a bridging oligo may be about 5-50, 50-100, 100-200, 200-300, 300-400, or 400-500 base pairs in length. In some cases, a bridging oligo may be at least about 5, 10, 15, 20, 25, 30, 35, 40, 45, 50, 55, 60, 65, 70, 75, 80, 85, 90, 95, 100, 125, 150, 175, 200, 300, 400 or 500 base pairs in length. In some cases, a bridging oligo may be at most about 5, 10, 15, 20, 25, 30, 35, 40, 45, 50, 55, 60, 65, 70, 75, 80, 85, 90, 95, 100, 125, 150, 175, 200, 300, 400 or 500 base pairs in length.

In some cases, a bridging oligo may also contain additional sequences. In some cases, a bridging oligo may be a degenerate primer or contain degenerate priming sequences. In some cases, a bridging oligo may comprise universal priming sequences.

In some cases, the bridging oligonucleotide may anneal to a region spaced any suitable distance from either the first or second probe in the locus of interest. In some cases, a bridging oligo may be directly adjacent to either the first or second probe, such that there is no space between the oligo and either the first probe or second probe. In some cases, the space found between the bridging oligo and either the first probe or second probe may be at least about 1 bp, 2 bp, 3 bp, 4 bp, 5 bp, 10 bp, 15 bp, 20 bp, 25 bp, 30 bp, 35 bp, 40 bp, 45 bp, 50 bp, 75 bp, 100, bp 125 bp, 150 bp, 175 bp, 200 bp, 300 bp, 400 bp, 500 bp, 600 bp, 700 bp, 800 bp, 900 bp, 1 Kb, 2 Kb, 3 Kb, 4 Kb, 5 Kb, or 10 Kb in length. In some cases, the space found between the bridging oligo and either the first or second probe may be at most about 1 bp, 2 bp, 3 bp, 4 bp, 5 bp, 10 bp, 15 bp, 20 bp, 25 bp, 30 bp, 35 bp, 40 bp, 45 bp, 50 bp, 75 bp, 100, bp 125 bp, 150 bp, 175 bp, 200 bp, 300 bp, 400 bp, 500 bp, 600 bp, 700 bp, 800 bp, 900 bp, 1 Kb, 2 Kb, 3 Kb, 4 Kb, 5 Kb, or 10 Kb in length.

One or more steps to remove unbound bridging oligo may be used. In some cases, this step may comprise a wash step. In other cases this may comprise an affinity purification step or combination thereof.

A contiguous ligation product may be formed with any suitable method. In one configuration, wherein no space exists between the bridging oligo and either the first or second probe, the bound oligonucleotides may be ligated together in one step. In an alternative configuration of the systems and methods of the disclosure, the bridging oligo may anneal to a complementary region that is not directly adjacent to either the first or second probe. In certain cases, an extension step may be performed, such that a polymerase and dNTPs may be used to extend sequences from the bridging oligo, first or second probe or combination thereof. In some cases, the polymerase and dNTPs may be used to fill the gap between the first probe and the bridging oligo, the bridging oligo and the second probe or a combination thereof. Following extension, the bound oligos may be ligated to form a contiguous polynucleotide product that spans a locus or locus of interest.

Additionally, use of polymerase in primer extension and hybridization of the first probe, second probe or one or more bridging oligos may be combined in one reaction mixture. In other cases, one or more steps may be performed sequentially.

### IX. Isolation of Bound Probes

After ligation, it is generally preferred to separate bound probes from unbound probes. In one configuration, bound probes may be affinity purified, using a combination of binding partners. In one example, probes, sequences linked to probes (i.e. bridging oligos), primer extension products, or adapter sequence ligated to the sample polynucleotide strands, may contain a binding partner such as biotin. The binding partner may then be used as bait for an additional binding partner, such as streptavidin, in an affinity purification step. In some cases, bound probes may be affinity purified from unbound probes. In other cases, sample polynucleotide strands, comprising a binding partner and bound probes may be affinity purified from unbound probes.

Generally, any chemical means for capture of the bound probes may be suitable. In some cases, capture may be achieved through methods comprising biotin and streptavidin, or streptavidin derivatives. For example, one embodiment of the disclosure provides for capture of polynucleotides, wherein biotinylated nucleotides may be incorporated during a primer extension step or a subsequent amplification step. In some cases, a mixture of nucleotides and biotinylated nucleotides may be used, wherein the mixture may be at least 1%, 2%, 5%, 10%, 20%, 30%, 40%, 50%, 60%, 70%, 80%, 90% or 100% biotinylated nucleotides. In other cases, the mixture may be at most 1%, 2%, 5%, 10%, 20%, 30%, 40%, 50%, 60%, 70%, 80%, 90% or 100% biotinylated nucleotides.

In alternative configurations of the systems and methods of the disclosure, unbound probes may be degraded by an exonuclease. For examples, after ligation, MIP or padlock probes become fully circularized, while unbound probes, which do not become contiguous, remain linear. Addition of exonuculease, such as exonuclease I, II, or III, may be used to selectively degrade linear polynucleotides, such as unbound polynucleotide probes, while fully circularized probes remain unaffected. After exonuclease treatment, bound circular probes may be eluted from the sample polynucleotide. In some cases elution may be performed via optional wash steps, such as with salt. In other cases bound probes may be melted off the sample polynucleotide strand through thermal means (i.e. raising the temperature of the reaction mixture), and as known in the art.

### X. Identification and Enumeration of Sequences

The methods of this disclosure generally provide for isolation of the ligation product and identification and enumeration of one or more sequences in the ligation product. In some cases, the identification and enumeration of sequences involve barcode sequences. In other cases, the identification and enumeration of sequences involves other sequences such as universal adapter sequences, universal priming sequences, linker sequences, portions of probe or hybridization sequence or in some cases a combination of sequences thereof.

Generally, the methods of this disclosure provide for one or more selective amplification steps, wherein in sequence specific primers may be used to amplify or enrich specific sequences in target molecules (i.e. ligation products or probes). In some cases, amplification is specifically directed to barcode sequences with barcode specific primers. In some cases, barcodes may be amplified with universal primers, designed to flank the barcode sequence.

After amplification, a variety of methods may be used to identify and enumerate sequences, including methods such as sequencing, quantitative PCR (qPCR) and other quantitative methods known in the art. The methods of this disclosure are particularly suitable for massively parallel sequencing of various selectively amplified sequences, such as barcodes, wherein sequences may be both identified and enumerated.

Generally, the method and system of this disclosure may utilize the systems and methods of US patent US 7,537,897 in using molecular barcodes to count molecules.

### A. Direct Enumeration of Ligation Products

In some instances of this disclosure, it may be suitable to directly enumerate and identify ligation products. In some cases, techniques with suitable sensitivity and selectivity may be used. In some cases, direct enumeration and identification of ligation products may involve direct sequencing with methods known in the art, as described herein. Sequences of the ligation products may be used to identify probe sequences, barcodes, adapter sequences, universal priming sequences, linker sequences or combination thereof. Sequences may also be enumerated based on sequence read counts.

In some cases, one or more quantifiable hybridization probes may bind to the ligation product at various sites. In some cases, the probe may be designed to anneal to one or more probe or hybridization sequences, barcode sequences, adapter sequences, linker sequences or combination thereof. In some cases, the probe sequence may be further attached to a fluorophore or fluorescent signal which may be quantified and correlated with the number of probe sequences present in the test sample. For example, certain products in the art, such as the Nanostring nCounter system may be used to enumerate probe counts, using a system involving a DNA hybridization probe conjugated to a series of fluorescent barcodes comprising nanoparticles. The system utilizes the nanoparticle barcodes to count probes with high sensitivity. Further, in other cases, additional probes hybridized to ligation products, may also be directly sequenced with methods described herein, and enumerated based on sequencing read counts.

In some cases, wherein the quantity of ligation products is not of sufficient quantity, selective amplification may be also be performed using the single stranded ligation products as a template. Primers may be designed to probe sequences, adapter sequences, barcodes, universal priming sites or combination thereof. Amplification strategies and primer set design methodologies are further described herein. In some cases, PCR products, amplified from the single stranded ligation products, may be used for identification and enumeration using various techniques including direct sequencing or other suitable quantitative methods.

### B. Second Strand Synthesis of Ligation Products

In some cases, where it may be preferable to obtain a double stranded ligation product, a subsequent second strand synthesis step may be performed as shown in **Fig. 4****.** In some cases a single primer, **428,** may be used to synthesize a strand, **424,** complementary to the single stranded ligation product. In this case, a single primer may anneal to a site on the ligation product, which may include probe sequences, barcode sequences, adapter sequences, universal primer sequences, linker sequences or combination thereof. In some cases, a polymerase is used to extend the complementary strand from the 3' end of the primer. In instances involving circular probes (i.e. MIP or padlock probes), the complementary strand may be ligated to the 5' end of the primer to create a circular double stranded polynucleotide.

Further, in some instances, one or more primers may be used. In some instances, at least about 1, 2, 3, 4, 5, 6, 7, 8, 9 or 10 primers may be used for second strand synthesis. In some instances at most about 1, 2, 3, 4, 5, 6, 7, 8, 9 or 10 primers may be used for second strand synthesis. In cases involving multiple primers, one or more primer extension products may be ligated together to form the second strand.

In some cases, one or more primers may also contain additional sequences, which may not be complementary to the single stranded ligation product. These additional sequences may include but are not limited to sequencer tail sequences, **420,** adapter sequences or barcode sequences and may be used for subsequent downstream steps. For example, in certain methods of this disclosure, a sequencer specific tail sequence may be incorporated into the primer used for second strand synthesis. After synthesis, the sequencer specific tail sequence may exist as a single stranded region in the double stranded molecule. This tail sequence may be designed to be compatible with various sequencing platforms, such as provided by Illumina. In this example, the tail sequence may be used to hybridize or capture the double stranded molecules onto a solid support for sequencing, as known in the art. These methods may be particularly useful in multiplex applications, wherein a plurality of molecules or probe sets are processed and sequenced in parallel.

In other cases, double stranded products may be directly sequenced using other methods known in the art and as described elsewhere in this disclosure. Direct sequencing may be performed to identify sequences in the ligation products and enumerate sequences as described herein.

### C. Cleavage of Ligation Products

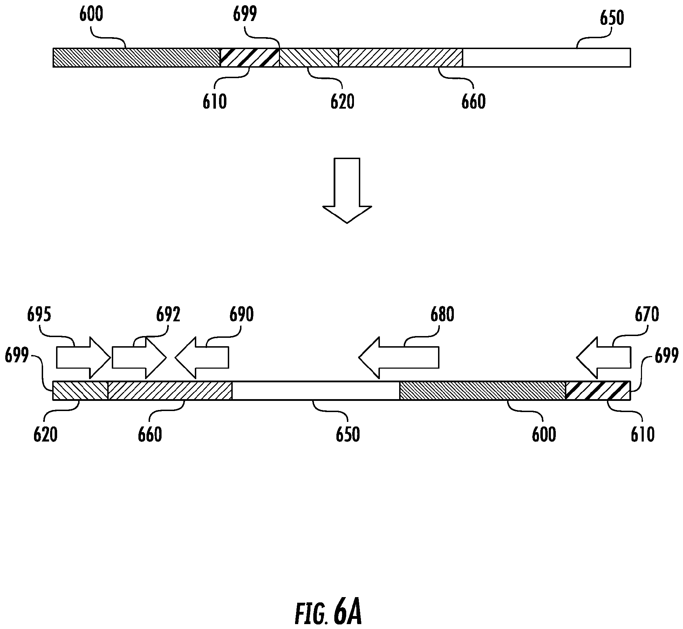

In some cases, it may be preferable to further cleave the contiguous ligation product, after second strand synthesis. In cases involving MIP probes, one configuration, as shown in **Fig. 6A** provides a MIP probe comprising a first probe, **600,** and second probe, **650,** flanking a set of universal priming sites, **610** and **620,** and a barcode sequence, **660.** A restriction site, **699,** may be incorporated into the probe, wherein the restriction site falls between two universal priming sites, **610** and **620.** After ligation, the first probe, **600,** and second probe, **650,** are joined together, at site **695.** After cleavage at restriction site **699,** the universal priming sites are physically separated and now flank both the barcode sequence and the now contiguous probe or hybridization sequences. This configuration provides for multiple amplification or sequencing strategies as further described herein.

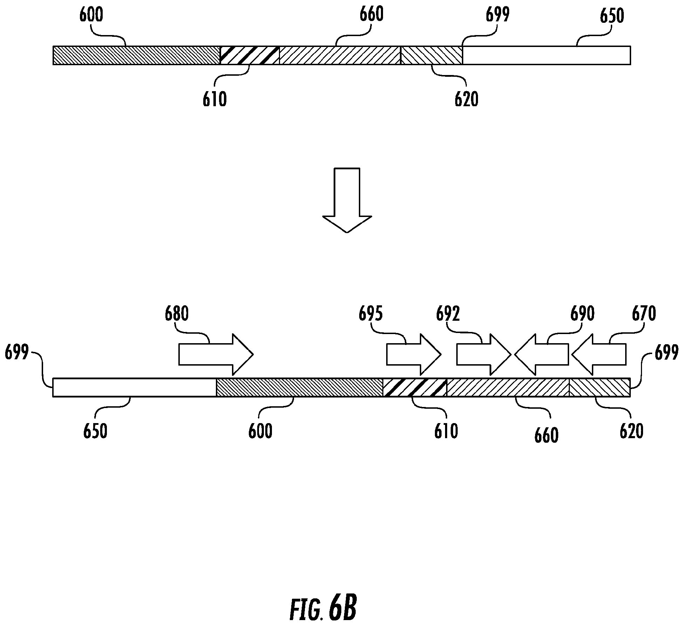

In another alternative configuration involving MIP probes, as shown in **Fig. 6B****,** a first probe, **600,** and second probe, **650,** flank a set of universal priming sites, **610** and **620,** which flank a barcode sequence, **660.** A restriction cleavage site, **699,** may be incorporated into the probe, wherein the cleavage site falls between one universal amplification site, **620** and the second probe sequence, **650.** After ligation, the first probe, **600,** and second probe, **650,** are ligated together, at site **695.** After cleavage at restriction site **699,** the universal priming sites flanking the barcode sequence remain and are now positioned adjacent to contiguous probe or hybridization sequences after restriction cleavage. This configuration provides for multiple amplification or sequencing strategies as further described herein.

### D. Selective and Universal Amplification of Ligation Products

### i. Selective Primer Set Design

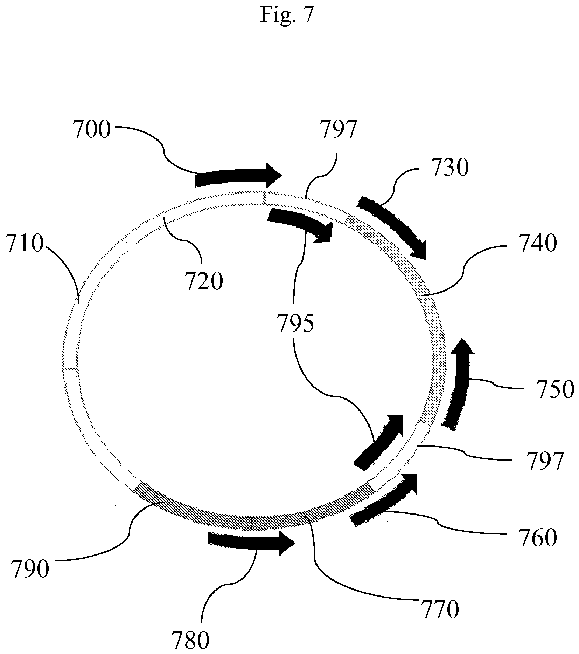

Generally, the methods of this disclosure provide for various strategies for amplification. In the case of selective amplification, primer sets may be designed in a variety of different ways. In some cases, primers may be designed to anneal to probe sequences, adapter sequences, universal priming sites, linker sequences or any combination thereof, as shown in **Fig. 6A****,** **6B** and **Fig. 7****.** Examples of different selective amplification strategies are provided for exemplary purposes and are not limiting. For example, in some cases, in which MIP probes are cleaved to form a configuration as shown in **Fig. 6A****,** a forward primer may be complementary to probe or hybridization sequences, **680.** The primer may be complementary to a portion of the ligated sequences, or to either the first or second probe hybridization sequences. This primer may be used in combination with a reverse primer complementary to a barcode sequence, **692** or a universal priming site **695.** As show in **Fig. 6B****,** a probe specific primer, complementary to one hybridization sequence or a combination of both sequences may be used as a reverse primer, **680.**

In another example, involving a circular ligation product that is not cleaved, as shown in **Fig. 7****,** selective primers may include a primer, **780,** complementary to probes sequences, **790** and **770,** or a primer, **700** complementary to additional linker sequences in the probe such as **720** or **710.**

In some cases, individual primers may be designed to anneal to one or more portions of various sequences as described herein. For example, in some cases a primer may be designed with a general structure, 5'MMMMMNNNNN, wherein M is a variable length region complementary to a sequence including but not limited to first and/or second probe sequence, adapter sequence, universal priming sequence, or linker sequences and N is selected from a different sequence from the same group. For example in **Fig. 7****,** a primer comprising sequence complementary to hybridization sequence and a universal amplification sequence may be used, **760.** In some instances, M may comprise sequence complementary to probe sequence and N comprises sequence complementary to barcode sequences. In some instances, M may comprise sequence complementary to a first probe sequence and N comprises sequence complementary to a second probe sequence. In some cases, the length, or number of nucleotides defined as either M or N may be at least about 1, 2, 3, 4, 5, 6, 7, 8, 9, 10, 15, 20, 25, 30, 35, 40, 45, 50, 75, 100, 125, 150, 175, 200, 300, 400 or 500 nucleotides. In some cases, the length, or number of nucleotides defined as either M or N may be at most about 1, 2, 3, 4, 5, 6, 7, 8, 9, 10, 15, 20, 25, 30, 35, 40, 45, 50, 75, 100, 125, 150, 175, 200, 300, 400 or 500 nucleotides.

### ii. Universal Primer Design

In some configurations of the methods the disclosure, ligation products, or selective amplification products, which may or may not contain hybridization sequences from the locus of interest, may be universally amplified. Universal amplification may be generally described as the use of universal primers to amplify one or more regions. Universal primers are priming sites common to one or more probes. Universal amplification products may be performed before or after one or more selective amplification steps. In some cases, universal amplification may be performed with universal primers complementary to universal priming sequence sites in a ligation product. In some cases, universal amplification may include primers complementary to barcode sequences. In other cases, universal priming sequence may be added to polynucleotides during second strand synthesis, or one or more selective amplification steps (i.e. primers for selective amplification sequences may be added to the polynucleotide regions during the selective amplification process), or universal adapter sequences, containing universal priming sites, may be added to regions flanking the sequence to be amplified (i.e. probe sequence, barcode sequence etc...) through methods such as adapter-ligation.

In some configurations, a selective round of amplification is performed. In some cases, selective amplification may involve amplification of probe sequences, adapter sequences, barcode sequences, linker sequences or any combination thereof. After selective amplification, universal amplification may be performed on selective amplification products, using primers set design strategies as described herein. In some cases, universal amplification may be performed on ligation products, using universal primers, followed by selective amplification of universal amplification products.

In one configuration of the systems and methods of the disclosure, universal amplification may be performed from universal amplification sites, common to one or more probes sets. Generally, all probes sets may comprise universal priming sites. A universal amplification step is generally preferred with the systems and methods of this disclosure, as this technique allows for the minimization of variation of amplification products. With a universal primer set, amplification across probe sets may be more uniform, as compared to selective amplification of individual probe sets using probe set specific primers. In some cases involving multiple probe specific primer sets, amplification inefficiencies may skew or alter amounts of amplification products, which, if enumerated in later steps, may affect calculations for copy numbers at specific loci. Further, the use of relatively few primers may also aid in providing a high throughput, simplified, single assay work flow.

In one configuration of the systems and methods of the disclosure, as shown in **Fig. 6A** and **6B****,** universal amplification may be performed using universal amplification primers, **670** and **695.** In this configuration, the amplification product may comprise the barcode sequence, **660,** and hybridization sequences (or sequences pertaining to the locus of interest). This may be useful for downstream applications in which amplification products may be sequenced and loci determined from sequencing data. Generally, a portion of the probe or sequences used for hybridization may be amplified. In some cases about 1%-10%, 20%-30%, 30%-40%, 40%-50%, 50%-60%, 60%-70%, 70%-80%, 80%-90%, 90%-99%, or 99%-99.99% of hybridization sequences may be amplified. In some cases at least about 1%, 2%, 3%, 4%, 5%, 6%, 7%, 8%, 9%, 10%, 20%, 30%, 40%, 50%, 60%, 70%, 80%, 90%, 95%, 99%, 99.9%, 99.99% of hybridization sequences may be amplified. In some cases at most about 1%, 2%, 3%, 4%, 5%, 6%, 7%, 8%, 9%, 10%, 20%, 30%, 40%, 50%, 60%, 70%, 80%, 90%, 95%, 99%, 99.9%, 99.99% of hybridization sequences may be amplified.

In other variations of this configuration, the barcode sequence may also be amplified using barcode specific primers, **690** and **692.** In some cases of the systems and methods of this disclosure, in which barcodes may be commonly assigned probe sets designed for related loci (as described herein), assignment of the barcode to known loci will have been predetermined. Amplification of the barcode sequence may also be achieved with different primer combinations, such as that with hybridization sequence specific primers, or other universal amplification primers.

In one particular case, as shown in **Fig. 6B****,** amplification of the barcode sequence may be achieved with universal primers, without the amplification and subsequent detection (i.e. sequencing) of hybridization sequences. Since the universal primer sites are designed to flank the barcode sequence, and remain intact after cleavage, these sites may be used to generate amplification products containing only the barcode sequence and sequences other than hybridization sequences.

In another configuration of the systems and methods of the disclosure, as shown in **Fig. 7****,** universal amplification may be performed using universal amplification primers, **795,** from a circular ligation product. In this configuration, the amplification product may comprise the barcode sequence, **740,** and universal priming sequences, **797,** in the amplification product. Barcode specific sequences may also be used, **730** and **750,** or a combination thereof. Hybridization sequences (or sequences pertaining to the locus of interest). This may be useful for downstream applications in which amplification products may be sequenced and loci determined from sequencing data. In other variations of this configuration, the barcode sequence may also be amplified using barcode specific primers, **690** and **692.**

### E. Amplification Techniques

Numerous amplification methods and techniques are known in the art. Any suitable methods may be used in the methods of this disclosure, so as to increase the quantity or amount of polynucleotides, while maintaining the initial content of sequence information of the original sample or ligation product. One or more amplification methods may be used and in one or more combinations.

Examples of amplification methods may include but are not limited to, polymerase chain reaction (PCR) (U.S. Pat. Nos. 4,683,195; and 4,683,202; PCR Technology: Principles and Applications for DNA Amplification, ed. H. A. Erlich, Freeman Press, NY, N.Y., 1992), ligase chain reaction (LCR) (Wu and Wallace, Genomics 4:560, 1989; Landegren et al., Science 241:1077, 1988), strand displacement amplification (SDA) (U.S. Pat. Nos. 5,270,184; and 5,422,252), transcription-mediated amplification (TMA) (U.S. Pat. No. 5,399,491), linked linear amplification (LLA) (U.S. Pat. No. 6,027,923), and the like, self-sustained sequence replication (Guatelli et al., Proc. Nat. Acad. Sci. USA, 87, 1874 (1990) and WO90/06995), selective amplification of target polynucleotide sequences (U.S. Pat. No. 6,410,276), consensus sequence primed polymerase chain reaction (CP-PCR) (U.S. Pat. No. 4,437,975), arbitrarily primed polymerase chain reaction (AP-PCR) (U.S. Pat. Nos. 5,413,909, 5,861,245) and nucleic acid based sequence amplification (NASBA). (See, U.S. Pat. Nos. 5,409,818, 5,554,517, and 6,063,603). Other amplification methods that may be used include: Qbeta Replicase, described in PCT Patent Application No. PCT/US87/00880, isothermal amplification methods such as SDA, described in Walker et al., Nucleic Acids Res. 20(7): 1691-6 (1992), and rolling circle amplification, described in U.S. Pat. No. 5,648,245. Other amplification methods that may be used are described in, U.S. Pat. Nos. 5,242,794, 5,494,810, 4,988,617 and in US6582938 and US Pub. No. 20030143599. In some aspects DNA is amplified by multiplex locus-specific PCR. In a preferred aspect the DNA is amplified using adaptor-ligation and single primer PCR. Other available methods of amplification, such as balanced PCR (Makrigiorgos, et al., Nature Biotech, 20:936-9 (2002)) and isothermal amplification methods such as nucleic acid sequence based amplification (NASBA) and self-sustained sequence replication (Guatelli et al., PNAS USA 87:1874 (1990)). Based on such methodologies, a person skilled in the art readily can design primers in any suitable regions to be amplified.

### F. Amplification Products and Conditions

In general, any suitable amplification products and conditions to produce products may be used in the methods of this disclosure. Various amplification lengths, cycle times, hybridization, annealing and extension conditions may be used, as appropriate for various amplification techniques and sequences.

### i. Amplification Lengths

Generally, the length of an amplified product may be any length and contain any sequence that may be useful in the enumeration of sequences. Generally, an amplified polynucleotide may be at least about 5 bp, 10 bp, 20 bp, 30 bp, 40 bp, 50 bp, 60 bp, 70 bp, 80 bp, 90 bp, 100 bp, 200 bp bp, 300 bp, 400 bp, 500 bp, 600 bp, 700 bp, 800 bp, 900 bp, 1 kb, 2 kb, 3, kb, 4 kb, 5 kb, 6 kb, 7 kb, 8 kb, 9 kb, 10kb, 20 kb, 30 kb, 40 kb, 50 kb, 75 kb, or 100 kb. Generally, an amplified polynucleotide may be at most about 5 bp, 10 bp, 20 bp, 30 bp, 40 bp, 50 bp, 60 bp, 70 bp, 80 bp, 90 bp, 100 bp, 200 bp bp, 300 bp, 400 bp, 500 bp, 600 bp, 700 bp, 800 bp, 900 bp, 1 kb, 2 kb, 3, kb, 4 kb, 5 kb, 6 kb, 7 kb, 8 kb, 9 kb, 10kb, 20 kb, 30 kb, 40 kb, 50 kb, 75 kb, or 100 kb.

### ii. Amplification Conditions

In general any suitable amplification conditions may be used, for either selective or universal amplification. In some cases, amplification may be linear. In some cases, amplification may be logarithmic. Since the methods of the disclosure provide for enumeration of one or more sequences, which may be amplified, it may be suitable to control amplification in various steps to control variability between samples.

For example, in some cases, a limited number of amplification cycles may be used in either a selective or universal amplification step. This may be particularly suitable for selective amplification wherein different primer sets for different loci or barcodes may behave differently under multiplex conditions wherein a plurality of loci or barcodes are used. Primers in different primer sets may differ in their ability to hybridize to template, and thus yield differences in amplification efficiency between primer sets. Each set of primers for a given locus may behave differently based on sequence context of the primer and sample DNA, buffer conditions, and other conditions. A universal DNA amplification for a multiplexed assay system may generally introduce less bias and variability.

To minimize amplification variation between one or more loci or barcodes, for example, amplification may be performed using a linear amplification method, followed by logarithmic universal amplification. In some cases, the number of cycles is limited between 1-50 cycles, such that amplification is linear or near linear. In some cases amplification cycles for linear amplification may be at least about 1, 2, 3, 4, 5, 6, 7, 8, 9, 10, 20, 30, 40 or 50 cycles. In some cases amplification cycles for linear amplification may be at most about 1, 2, 3, 4, 5, 6, 7, 8, 9, 10, 20, 30 ,40 or 50 cycles. In some cases, after linear selective amplification of sequences from ligation products, a logarithmic universal amplification step may be performed as described herein. Universal amplification, wherein common primer sets may be used for a plurality of loci or barcode amplification products may further reduce amplification variability, while producing increasing amounts of sample.

In other cases, logarithmic amplification may be used before linear amplification. In some cases amplification cycles for logarithmic amplification may be at least about 1, 2, 3, 4, 5, 6, 7, 8, 9, 10, 20, 30 ,40 or 50 cycles. In some cases amplification cycles for logarithmic amplification may be at most about 1, 2, 3, 4, 5, 6, 7, 8, 9, 10, 20, 30 ,40 or 50 cycles.

Generally, any suitable number of primer sets may be used for amplification. In some cases, amplification primer sets may be about equal to the number of loci tested. In some cases, primers sets may be at least about 1, 2, 3, 4, 5, 6, 7, 8, 9, 10, 20, 30, 40, 50, 60, 70, 80 , 90 100, 125, 150, 175, 200, 300, 400, 500, 600, 700 800, 900 or 1000 primer sets. In some cases, primers sets may be at most about 1, 2, 3, 4, 5, 6, 7, 8, 9, 10, 20, 30, 40, 50, 60, 70, 80, 90 100, 125, 150, 175, 200, 300, 400, 500, 600, 700 800, 900 or 1000 primer sets.

### G. Sequence Identification and Enumeration Methods

In general, any method or technique for barcode sequence identification and enumeration of barcodes may be used with the systems and methods of this disclosure. In some cases identification may be performed using PCR, as described herein, whereby sequences may be determined directly from the presence or absence of amplification products and further quantified. In some cases, identification of sequences may be performed through sequencing. Generally, enumeration of sequences may be performed using any suitable quantitative methods, including but not limited to qPCR, hybridization methods and sequencing or combination thereof.

### i.PCR Methods

In some cases, methods involving PCR may be used to identify and enumerate sequences. In the case of identification, in some cases, successful amplification products with primers containing probe sequences may indicate the presence of a particular locus. In some cases, failed amplification of products of probe sequences may indicate the absence of a particular locus. In other cases, probes may be associated with an individual unique identifier sequences, or barcode. PCR amplification may be performed on the barcode alone, or a combination of probe sequences and barcode. In some cases, before testing a sample, a particular barcode of known identity is assigned to one or more probe sets, complementary to regions of a known locus. In some cases, successful amplification products of barcode sequences may indicate the presence of a particular locus. In some cases, failed amplification of products of a barcode sequence may indicate the absence of a particular locus.

In some cases, PCR amplification products may be quantified using any suitable method. In some cases, methods may be quantitative PCR (qPCR) or variations thereof. In some cases, this may be a fluorescence based approach, wherein fluorescence signal may be quantified. Quantified signal may be used to calculate a relative abundance of original template (i.e. ligation product) and thus provide information relating to the relative abundance of a particular locus. In some cases, relative abundance measurements may be used in determining the presence or absence of genetic alterations such as CNV.

### ii. Hybridization Methods

Alternatively, in another configuration of this disclosure, the entire length, or a portion of the amplification product may be analyzed using hybridization techniques. Methods for performing polynucleotide hybridization assays for detection are known in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y., 1989); Berger and Kimmel Methods in Enzymology, Vol. 152, Guide to Molecular Cloning Techniques (Academic Press, Inc., San Diego, Calif., 1987); Young and Davis, P.N.A.S, 80: 1194 (1983). Methods and apparatus for carrying out repeated and controlled hybridization reactions have been described in U.S. Pat. Nos. 5,871,928, 5,874,219, 6,045,996 and 6,386,749, 6,391,623.

In the case of identification, successful hybridization of additional probes to sequences containing probe sequences of the original ligation products may indicate the presence of a particular locus. In some cases, failed hybridization of additional probes to probe sequences of the original ligation products may indicate the absence of a particular locus. In other cases, probes of the original ligation products may be associated with an individual unique identifier sequence, or barcode.

Hybridization may be performed on the barcode alone, or a combination of the original probe and barcode. In some cases, before testing a sample, it is known, which probe sequence, and thus which locus, may be associated with a particular barcode. In some cases, successful hybridization of probes to barcode sequences may indicate the presence of a particular locus. In some cases, failed hybridization of probes to a barcode sequence may indicate the absence of a particular locus.

The present disclosure also provides for signal detection of hybridization between ligands in certain preferred aspects. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in US8391582 and WO99/47964.

In some cases, hybridization probes may be quantified to enumerate sequences. In some cases, probes are conjugated to a chemical agent, fluophore or ligand which may be quantified. In one particular example, the methods of this disclosure may be suitable with a counting system such as provided by the Nanostring nCounter system.

Methods and apparatus for signal detection and processing of intensity data are disclosed in, for example, U.S. Pat. Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in US8391582 and WO99/47964.

### iii. Sequencing Methods

Numerous methods of sequence determination are compatible with the systems and methods of the disclosures. Exemplary methods for sequence determination include, but are not limited to, hybridization-based methods, such as disclosed in Drmanac, U.S. Pat. Nos. 6,864,052; 6,309,824; and 6,401,267; and Drmanac et al, U.S. patent publication 2005/0191656, sequencing by synthesis methods, e.g., Nyren et al, U.S. Pat. Nos. 7,648,824, 7,459,311 and 6,210,891; Balasubramanian, U.S. Pat. Nos. 7,232,656 and 6,833,246; Quake, U.S. Pat. No. 6,911,345; Li et al, Proc. Natl. Acad. Sci., 100: 414-419 (2003); pyrophosphate sequencing as described in Ronaghi et al., U.S. Pat. Nos. 7,648,824, 7,459,311, 6,828,100, and 6,210,891; and ligation-based sequencing determination methods, e.g., Drmanac et al., U.S. Pat. Appl. No. 20100105052, and Church et al, U.S. Pat. Appin Nos. 20070207482 and 20090018024.

Sequence information may be determined using methods that determine many (typically thousands to billions) nucleic acid sequences in an intrinsically parallel manner, where many sequences are read out preferably in parallel using a high throughput serial process. Such methods include but are not limited to pyrosequencing (for example, as commercialized by 454 Life Sciences, Inc., Branford, Conn.); sequencing by ligation (for example, as commercialized in the SOLiD^{™} technology, Life Technology, Inc., Carlsbad, Calif.); sequencing by synthesis using modified nucleotides (such as commercialized in TruSeq^{™} and HiSeq^{™} technology by Illumina, Inc., San Diego, Calif., HeliScope^{™} by Helicos Biosciences Corporation, Cambridge, Mass., and PacBio RS by Pacific Biosciences of California, Inc., Menlo Park, Calif.), sequencing by ion detection technologies (Ion Torrent, Inc., South San Francisco, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

Further, sequences reads may be enumerated through quantifying the number of reads per locus or barcode.

### XI. Read Counts and Filtering

In certain cases, it may be suitable to filter or select read count data before determination of genetic alterations. Generally, measuring the abundance for each locus of interest, or a subset of the loci of interest, may be used to determine the presence or absence of a genetic alteration.

There are many standard methods for choosing the subset of loci of interest. These methods include outlier exclusion, where the loci of interest with detected levels below and/or above a certain percentile are discarded from the analysis. In some cases, the percentile may be at least about the lowest and highest 1%, 2%, 3%, 4%, 5%, 6%, 7%, 8%, 9% 10%, 11%, 12%, 13%, 14%, 15%, 16%, 17%, 18%, 19%, 20%, 21%, 22%, 23%, 24%, or 25%, as measured by abundance. In some cases, the percentile may be at most about the lowest and highest 1%, 2%, 3%, 4%, 5%, 6%, 7%, 8%, 9% 10%, 11%, 12%, 13%, 14%, 15%, 16%, 17%, 18%, 19%, 20%, 21%, 22%, 23%, 24%, or 25%, as measured by abundance.

Another method for choosing the subset of selected loci includes the elimination of regions that fall outside of some statistical limit. For instance, loci of interest that fall outside of one or more standard deviations of the mean abundance may be removed from the analysis.

In some cases, a subset of loci of interest may be used to compare the relative abundance of a selected locus to the expected abundance of the same selected locus in a healthy or normal sample and discard any loci of interest that fail the expectation test.

To further minimize the variation in the systems and methods of the disclosure, the number of times each locus of interest is measured may be increased. As described herein, in contrast to the random methods of detecting genetic alterations where the genome is measured on average less than once, the systems and methods of this disclosure may test each locus of interest multiple times. Generally, when counting events, the variation in the counting may be determined by Poisson statistics, and the counting variation may be generally equal to one divided by the square root of the number of counts.

In some cases, loci of interest are each measured on average at least about 1, 2, 3, 4, 5, 6, 7, 8, 9, 10, 15, 20, 30, 40, 50, 60, 70, 80, 90, 100, 200, 300, 400, 500, 600, 700, 800, 900, 1000, 2000, 3000, 4000 or 5000 times. In some cases, loci of interest are each measured on average at most about 1, 2, 3, 4, 5, 6, 7, 8, 9, 10, 15, 20, 30, 40, 50, 60, 70, 80, 90, 100, 200, 300, 400, 500, 600, 700, 800, 900, 1000, 2000, 3000, 4000 or 5000 times.

In another configuration, subsets of loci of interest may be chosen randomly but with sufficient numbers of loci to yield a statistically significant result in determining whether a chromosomal abnormality exists. Multiple analyses of different subsets of loci of interest can be performed within a maternal sample to yield more statistical power. In this example, it may or may not be necessary to remove or eliminate any loci prior to the random analysis. For example, if there are 100 selected loci for chromosome 21 and 100 selected loci for chromosome 18, a series of analyses could be performed that evaluate fewer than 100 loci for each of the chromosomes.

In other cases, read counts for selected loci may be filtered or limited in counting, if amplification bias is detected. Internal references, standard controls and other quality control techniques may be independently employed to identify suspect loci for variability, such as due to amplification bias. In some cases, it may be preferable to limit read counts for suspect loci to prevent skewing of data.

### XII. Determination of Genetic Alterations in a Sample

After enumeration of sequence reads, and optional filtering, this disclosure provides methods for the determination of the presence or absence of a genetic alteration at one or more loci (i.e. CNV) in a sample comprising a mixture of fetal and maternal polynucleotides. In some cases, algorithms may be used to detect the presence or absence of genetic alterations. In some cases, algorithms may be used to generate a profile of potential genetic alterations. In some cases a profile may be indicative of the presence or absence of a CNV, as described herein, and may not conclusively indicate that the CNV is present or absent. A profile that is indicative of the presence or absence, respectively, of CNV may indicate an increased probability that a CNV is present or absent, respectively. In some cases a probability score or a degree of certainty may be provided for one or more genetic alterations (i.e. CNVs).

Alternatively the presence or absence of CNV may also be determined or confirmed by other systems and methods that may or may not rely on specific methods as described herein. In some cases, techniques such as PCR, qPCR or comparative genomic hybridization may be used alone or in combination with probe based approaches for detection of genetic alterations.

Determination of genetic alterations in a sample may be performed with a variety of suitable methods. In one example, for instance, an internal reference is used for comparison. In some cases, an internal reference is the use of a locus present in a "normal" abundance (e.g., disomy for an autosome) to compare against a locus present in putatively abnormal abundance, (i.e. duplication, aneuploidy et...), in the same sample. While the use of one such "normal" chromosome as a reference chromosome may be sufficient, it is also possible to use two or more normal chromosomes as the internal reference chromosomes to increase the statistical power of the quantification. In other cases, an external reference may be used, comprising one or more samples of a known genetic state (i.e. known copy number for one or more loci, euploidy, trisomy, etc...).

### A. Detecting Chromosomal Distributions

In some cases, an internal reference is used to calculate a ratio of abundance of the putatively abnormal chromosomes to the abundance of one or more normal chromosomes in a sample, called a chromosomal ratio. A ratio for individual chromosomes may also be calculated, in which the abundance or counts of each of the loci for each chromosome may be summed together to calculate the total counts for each chromosome. For chromosome ratios, the total counts for one chromosome are then divided by the total counts for a different chromosome to create a chromosomal ratio for those two chromosomes.

Alternatively, a chromosomal ratio for each chromosome may be calculated by first summing the counts of each of the loci for each chromosome, and then dividing the sum for one chromosome by the total sum for two or more chromosomes. Once calculated, the chromosomal ratio is then compared to the average chromosomal ratio from a normal population.

The average may be the mean, median, mode or other average, with or without normalization and exclusion of outlier data. In some cases, the mean is used. Chromosomal ratios from the normal population are calculated using the normal variation of the measured chromosomes. This variation may be expressed in different ways. In some cases it is expressed as the coefficient of variation, or CV. When the chromosomal ratio from the sample is compared to the average chromosomal ratio from a normal population, if the chromosomal ratio for the sample falls statistically outside of the average chromosomal ratio for the normal population, the sample may contain an aneuploidy. Statistical thresholds to determine an aneuploidy may be set depending upon the variation in the measurement of the chromosomal ratio and the acceptable false positive and false negative rates for the desired assay. In general, this threshold may be a multiple of the variation observed in the chromosomal ratio. For example, in some cases, this threshold is at least about 1, 2, 3, 4, 5, 6, 7, 8, 9, 10 times the variation of the chromosomal ratio. In some cases, this threshold is at most about 1, 2, 3, 4, 5, 6, 7, 8, 9, 10 times the variation of the chromosomal ratio.

In general, the chromosomal ratio may be determined by summing the counts of loci by chromosome. Generally, the same number of selected loci for each chromosome is used. An alternative method for generating the chromosomal ratio may be used to calculate the average counts for the loci for each chromosome. The average may be any estimate of the mean, median or mode, although generally an average is used. The average may be the mean of all counts or some variation such as a an adjusted or weighted average. Once the average counts for each chromosome have been calculated, the average counts for each chromosome may be divided by the other to obtain a chromosomal ratio between two chromosomes, the average counts for each chromosome may be divided by the sum of the averages for all measured chromosomes to obtain a chromosomal ratio for each chromosome as described herein.

As highlighted above, the ability to detect an aneuploidy in a maternal sample where the putative DNA is in low relative abundance depends greatly on the variation in the measurements of different selected loci in the assay. Numerous analytical methods can be used to reduce this variation and thus improve the sensitivity of this method to detect aneuploidy. One method for reducing variability of the assay is to increase the number of selected loci used to calculate the abundance of the chromosomes. In general, if the measured variation of a single selected locus of a chromosome is X%, and Y% selected loci are measured on the same chromosome, the variation of the measurement of the chromosomal abundance calculated by summing or averaging the abundance of each selected locus on that chromosome may be approximately as X% divided by Y%. The variation of the measurement of the chromosome abundance may be approximately the average variation of the measurement of each selected locus' abundance divided by the square root of the number of loci.

In some cases of this disclosure, loci counts may be determined by enumeration of associated barcodes. In some cases, barcode sequences may be identical for different loci on a similar chromosome. Barcodes may then be amplified and counted. In this case, variation in amplification and thus counting across loci of one chromosome may be minimized. In another example, loci of different chromosomes may be associated with a similar distribution of one or more barcodes. While variation in amplification efficiencies may exist for specific primer sets for the amplification of different barcodes, use of a similar distribution of primer sets across chromosomes may allow for minimization of amplification bias and variability across the sample. This may improve comparisons of counts, especially of one chromosome to another (i.e. chromosome ratios.)

### B. Locus-based CNV identification

In some cases, CNVs may be detected at one or more individual loci. In such cases, CNVs may be identified by detecting if particular loci are elevated above (i.e a gain in copy number) or decreased below (i.e. loss of copy number) a threshold or reference level. In some cases, particular loci may be known to be associated with a particular disease, disease state or infection. Comparison of various loci is determined by the number of read counts per locus, often referred to as depth of coverage. There are various programs or algorithms known in the art that may be used to identify CNVs by depth of coverage. In some cases an algorithm uses a normalized depth of coverage ratio to evaluate the relative read counts per locus as compared to the reference. In this case, the reference consists of median read counts for a plurality of loci obtained from a large dataset of "normal" (i.e. disomic) samples that have been generated in the same manner as the test sample. In other cases, a reference may comprise data from one or more known euploid genomes. In other cases, a reference may comprise data from one or more known samples with other defined genotypes, such as trisomic or monosomic loci. CNVs at one or more loci may be identified by a deviation from the standard distribution of copy number.

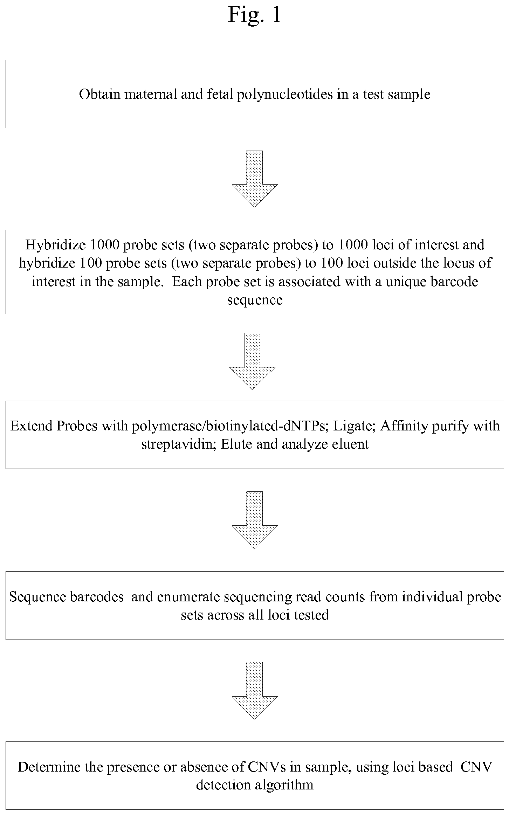

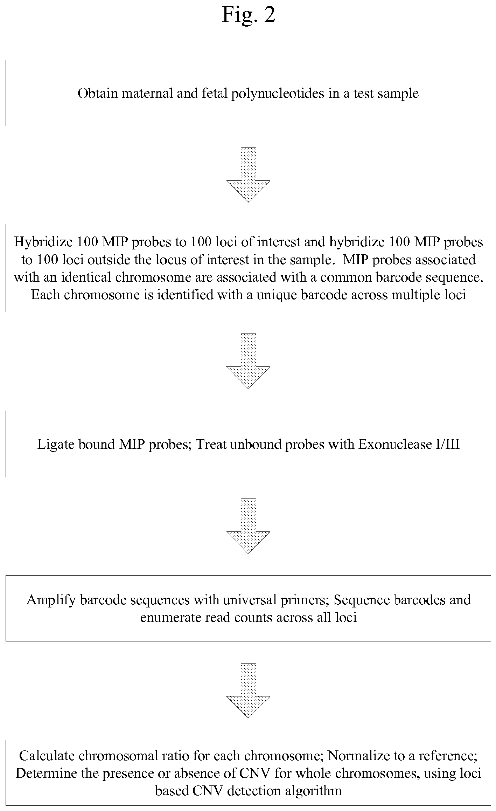

Any suitable algorithm that identifies CNVs using depth of coverage information may be used with the compositions and methods of this disclosure. Alternative algorithms may include but are not limited to programs such as CONTRA, XHMM, PennCNV, CoNIFER, VarScan, CNVSeq, cn.FARMS, BIC-seq and Console. Examples of work-flow related to loci based CNV detection may be found in Fig. **1** and **Fig. 2****.**

### C. Allele-based CNV identification

In some cases, CNVs may be identified by assessing the frequency of alleles from loci. In cases where there is a deletion, this region of sequence would have no heterozygous alleles, referred to as loss of heterozygosity (LOH). LOH may also arise if there is uniparental disomy (UPD), a situation in which both copies of a chromosome or segment of a chromosome is derived from the same parent. In the case of a trisomy, for heterozygous SNPs, one of the alleles may have a 2-fold increase, causing the ratios of the allelic expression to be shifted relative to the normal pattern. The effect of copy number gains on the allelic ratio may be smaller for gains than for losses. Any suitable algorithm may be used to detect such cases.

In some cases, probes may be designed to detect certain SNPs. In some cases, MIPS probes, or padlock probes, may be particularly suitable, wherein an SNP exists in the region between the first and second probe. Use of MIP probes for SNP detection is known in the art and may be detected using methods as described herein. In some cases, amplification of a region or a portion of a region containing a SNP between the first and second probe may be sequenced and analyzed. Read counts for individual SNP alleles may then be determined.

In some cases, an algorithm may evaluate the allele frequencies of heterozygous SNPs in the sample to determine if there is a deviation from the expected frequencies. For this analysis, the frequency of the non-reference allele 'X' may be determined for each polymorphic SNP by calculating by the number of reads of the X allele divided by the total number of reads or depth-of-coverage. The expected frequencies for the X allele frequencies for autosomes and the X chromosome in females is 0.5 and 0 or 1 for the X and Y chromosomes in male samples (the sex of a fetus may be determined by the presence/absence of Y-linked genes). To evaluate differences for polymorphic position, the expected frequencies are tested against other allele SNP frequencies. Segmentation of individual SNP data may then be performed using various statistical steps, such as circular binary segmentation (CBS). Generally, a test is performed to measure statistical significance between variance of sample frequency from that of reference frequency using an F-test for equality of variance. In some cases, this reference is composed of median X allele frequency values for the heterozygous SNPs present in the test sample from any of the reference types as describe herein. This segmentation process may identify contiguous regions in the genome in which there is skewed allelic expression in the sample relative to the reference, thereby identifying CNVs that may span more than one loci.

In some cases, CNVs may be detected by allele ratios. A SNP ratio that is either higher or lower when compared to a known value determined in the control sample, may indicate the presence of a CNV such as aneuploidy.

In some cases, an increase in the SNP ratio may be at least about 1%, 2%, 5%, 10%, 20%, 30%, 40%, 50%, 60%, 70%, 80%, 80%, or 100% higher as compared to the average value in the control sample, and may indicate the presence of a CNV. In some cases, an increase in the SNP ratio may be at most about 1%, 2%, 5%, 10%, 20%, 30%, 40%, 50%, 60%, 70%, 80%, 80%, or 100% higher as compared to the average value in the control sample, and may indicate the presence of a CNV.

In some cases, an increase in the SNP ratio may be at least about 1%, 2%, 5%, 10%, 20%, 30%, 40%, 50%, 60%, 70%, 80%, 80%, or 100% lower as compared to the average value in the control sample, and may indicate the presence of a CNV. In some cases, an increase in the SNP ratio may be at most about 1%, 2%, 5%, 10%, 20%, 30%, 40%, 50%, 60%, 70%, 80%, 80%, or 100% lower as compared to the average value in the control sample, and may indicate the presence of a CNV.

In some cases a SNP ratio may be measured in standard deviations higher or lower than the average value in the control sample and is indicative of an increased risk of there being a CNV. In some cases, a SNP ratio may be at least about 0.1, 0.2, 0.3, 0.4, 0.5, 0.6, 0.7, 0.8, 0.9, 1, 1.5, 2, 2.5, 3, 3.5, 4, 4.5 or 5 standard deviations higher than the average value in the control sample and may indicate presence of a CNV. In some cases, a SNP ratio may be at most about 0.1, 0.2, 0.3, 0.4, 0.5, 0.6, 0.7, 0.8, 0.9, 1, 1.5, 2, 2.5, 3, 3.5, 4, 4.5 or 5 standard deviations higher than the average value in the control sample and may indicate presence of a CNV.

In some cases, a SNP ratio may be at least about 0.1, 0.2, 0.3, 0.4, 0.5, 0.6, 0.7, 0.8, 0.9, 1, 1.5, 2, 2.5, 3, 3.5, 4, 4.5 or 5 standard deviations lower than the average value in the control sample and may indicate presence of a CNV. In some cases, a SNP ratio may be at most about 0.1, 0.2, 0.3, 0.4, 0.5, 0.6, 0.7, 0.8, 0.9, 1, 1.5, 2, 2.5, 3, 3.5, 4, 4.5 or 5 standard deviations lower than the average value in the control sample and may indicate presence of a CNV.

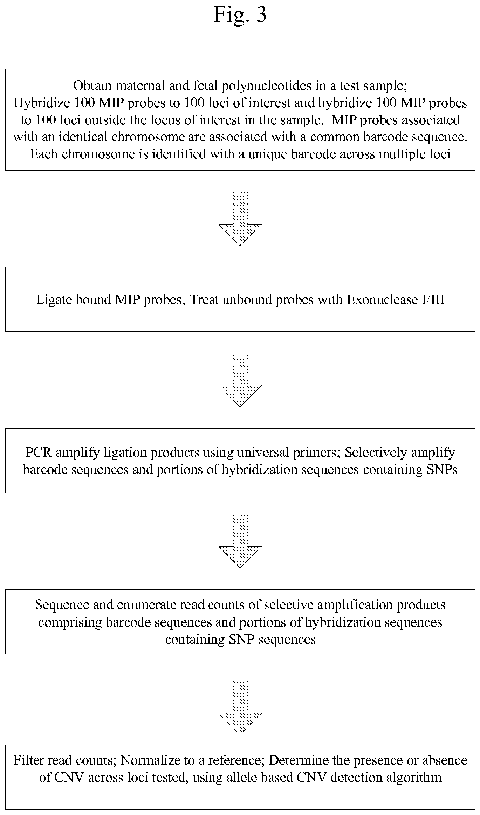

In some cases, CNVs, may be assessed using the depth of coverage approach applied to allelic expression. In such as case, the alleles may be divided into 2 groups, one corresponding to higher expressed alleles and one group corresponding to lower expressed alleles. Each group may then be compared as in a similar manner as locus read count data, as described herein. In some cases allele-level analysis may be preferred to locus-level analysis as the magnitude of the relative changes in copy number may be larger and consequently may result in larger read counts. An example of work-flow related to allele based CNV detection may be found in **Fig. 3****.**

### D. Number of Selected Loci

In some cases, the accuracy and resolution of the assay may improve with increasing number of loci tested. Increasing the number of loci, may also be particularly suitable for increasing the accuracy and resolution for call of CNVs toward whole chromosomes. The number of loci sampled per chromosome may be at least about 1, 2, 3, 4, 5, 6, 7, 8, 9, 10, 20, 30, 40, 50, 60, 70, 80, 90, 100, 200, 300, 400, 500, 600, 700 800, 900, 1000, 2000, or 5000 loci. he number of loci sampled per chromosome may be at most about 1, 2, 3, 4, 5, 6, 7, 8, 9, 10, 20, 30, 40, 50, 60, 70, 80, 90, 100, 200, 300, 400, 500, 600, 700 800, 900, 1000, 2000, or 5000 loci.

### E. Interpretation of Genetic Alterations

Following the identification of genetic alterations such as CNVs, the relevance of the genomic abnormality may be assessed to determine if it is likely pathogenic or benign. To determine the impact, databases that catalog genomic variants such as ENSEMBL (http://www.ensembl.org), the database of chromosomal imbalance and phenotype in humans using ensembl resources (DECIPHER, http://www.sanger.ac.uk/PostGenomics/decipher/). and the database of genomic variants (DGV http://proiects.tcag.ca/variation) may be consulted to determine if there may be phenotypic or health effects as a results of the genetic alteration.

Other considerations may include the size of the CNV and genomic content, detection of evidence of dosage sensitive genes in the online mendelian inheritance in man (OMIM) database (www.ncbi.nlm.nih.gov/omim) as well as review of current literature. Based on some or all of these analyses, an estimation of the likelihood of the pathogenicity of a CNV may be determined.

### XIII. Applications

### A. Fetal and Maternal Health

Generally, the systems and methods of this disclosure may be directed towards the assessment of the quality and health, of a mother or a fetus. Generally, the system and methods of this disclosure may be used to assess any appropriate disease or disease state associated with genetic alterations, such as CNVs. In some cases, this information may be used to support a decision regarding treatment of a mother or fetus. In other cases, this information may be used to support a decision regarding pregnancy (i.e. termination or continuation of pregnancy). In some cases involving IVF, information about genetic alterations in embryos may be used in selection decisions regarding which embryos to implant in a female.

In other cases, the systems and methods of this disclosure may be used to provide predictive information concerning relative health issues that may or may not arise in the fetus or after the fetus is born. In some cases, causal variants may be identified in either a mother or a fetus and used to determine or assess risk for the development of a particular disease.

### B. Early Detection of Cancer

The systems and methods provided herein may be used to monitor already known cancers, or other diseases in either a mother or fetus. This may allow either a mother, fetus or practitioner to adopt treatment options in accord with the progress of the disease. In this example, the systems and methods described herein may be used to construct genetic profiles of a particular subject of the course of the disease. In some instances, cancers can progress, becoming more aggressive and genetically unstable. In other examples, cancers may remain benign, inactive or dormant. The system and methods of this disclosure may be useful in determining disease progression.

Further, the systems and methods described herein may be useful in determining the efficacy of a particular treatment option. In one example, treatment options may actually affect the nature or amount of genetic alterations, such as CNVs. In some cases, monitoring of genetic alterations may be useful in monitoring of treatment efficacy. In some cases, this correlation may be useful in selecting or changing a therapy.

### C. Early Detection of Diseases Related to Numerical Chromosomal Abnormalities

In other applications, systems and methods of this disclosure may be used to detect other diseases associated with fetal or embryonic genetic alterations related to chromosomal abnormities.

For example, the systems and methods of this disclosure allow for detection of numerical abnormalities, such as those in which there is an extra set(s) of the normal (or haploid) number of chromosomes (triploidy and tetraploidy), those with a missing individual chromosome (monosomy) and those with an extra individual chromosome (trisomy and double trisomy). Approximately half of spontaneous abortions are associated with the presence of an abnormal number of chromosomes in the karyotype of the fetus, which makes aneuploidy the leading cause of miscarriage. Systems and methods of this disclosure may aid in early detection and possible treatment options for aneuploidy based disease states. Trisomy is the most frequent type of aneuploidy and occurs in 4% of all clinically recognized pregnancies. The most common trisomies involve the chromosomes 21 (associated with Down syndrome), 18 (Edward syndrome) and 13 (Patau syndrome). Other aneuploidies are associated with Turner syndrome (presence of a single X chromosome), Klinefelter syndrome (characterized by an XXY karyotype) and XYY disease (characterized by an XYY karyotype). The composition and systems and methods of the disclosure may be useful for detection of aneuploidy related afflictions including, but not limited to: Down syndrome, Edward syndrome and Patau syndrome, as well as Turner syndrome, Klinefelter syndrome and XYY disease.

### D. Early Detection of Diseases Related to X-linked diseases

The systems and methods of the disclosure may be used to detect chromosomal abnormalities involving the X chromosome. A large number of these chromosomal abnormalities are known to be associated with a group of diseases and conditions collectively termed X-linked disorders. For example, the systems and methods of the disclosure may be used to detect mutations in the HEMA gene on the X chromosome (Xq28), which are associated with Hemophilia A, a hereditary blood disorder, primarily affecting males and characterized by a deficiency of the blood clotting protein known as Factor VIII resulting in abnormal bleeding.

In another example, the systems and methods of this disclosure may also be used to detect an amplification (presence of more than 200 copies) of a CGG motif at one end of the FMR1 gene (Xq27.3) on the X chromosome, which is associated with Fragile X syndrome, the most common inherited form of mental retardation currently known.

### D. Early Detection of Diseases Related to Loci of Interest in Telomeres

In addition to Fragile X syndrome, a number of other retardation disorders are known to result from chromosomal abnormalities involving the terminal regions (or tips) of chromosomes (i.e., telomeres). A large portion of telomeric DNA is generally shared among different chromosomes. However telomeres also comprise a unique (much smaller) sequence region that is specific to each chromosome and is gene rich. Chromosome rearrangements involving telomeric regions can have serious clinical consequences. For example, submicroscopic subtelomeric chromosome rearrangements have been found to be a significant cause of mental retardation with or without congenital anomalies. Telomere regions have the highest recombination rate and are prone to aberrations resulting from illegitimate pairing and crossover. Since the terminal portions of most chromosomes appear nearly identical by routine karyotyping analysis at the 450- to 500-band level, detection of chromosomal rearrangements in these regions is difficult using standard methodologies. The systems and methods of this disclosure, which may provide for a much higher resolution than conventional karyotyping systems and methods, may be used to detect such subtelomeric rearrangements.

### E. Early Detection of other various CNV Diseases

In other examples, the systems and methods of the disclosure may be used to detect various other CNV related fetal diseases. These may include but are not limited to deletion of segment q11-q 13 on chromosome 15, which, when paternally derived from chromosome 15, is associated with Prader-Willi syndrome (a disorder characterized by mental retardation, decreased muscle tone, short stature and obesity). When maternally derived from chromosome 15, this genetic alteration is linked to Angelman syndrome (a neurogenetic disorder characterized by mental retardation, speech impairment, abnormal gait, seizures and inappropriate happy demeanor).

In another example, systems and methods of the disclosure may also be used to detect microdeletions in chromosome 22, for example those occurring in band 22q11.2, which are linked to DiGeorge syndrome, an autosomal dominant condition that is found in association with approximately 10% of cases in prenatally-ascertained congenital heart disease.

In another example, a segmental duplication of a subregion on chromosome 21 (such as 21q22), which can be present on chromosome 21 or another chromosome (i.e., after translocation) and is associated with Down syndrome may also be detected using the systems and methods of the disclosure.

### F. Early Detection of Immune Diseases, Infection, and Fetal Sex

Various other diseases and infections may result in other types of conditions that may be suitable for early detection and monitoring. For example, in certain cases, genetic disorders or infectious diseases may cause a certain genetic mosaicism within a subject. This genetic mosaicism may cause copy number variation and rare mutations that may be detected with the systems and methods of this disclosure. In another example, the system and methods of the disclosure may also be used to monitor the genomes of immune cells within the body of either the mother or fetus. Immune cells, such as B cells, may undergo rapid clonal expansion upon the presence certain diseases. Clonal expansions may be monitored using copy number variation detection and certain immune states may be monitored. In this example, copy number variation analysis may be performed over time to produce a profile of how a particular disease may be progressing.

Further, the systems and methods of this disclosure may also be used to monitor systemic infections themselves, as may be caused by a pathogen such as a bacteria or virus. Copy number variation or even rare mutation detection may be used to determine how a population of pathogens is changing during the course of infection. This may be particularly important during chronic infections, such as HIV/AIDs or Hepatitis infections, whereby viruses may change life cycle state and/or mutate into more virulent forms during the course of infection.

Further, the systems and methods of the disclosure may also be used to determine the sex (i.e. male or female) of a fetus. For example, probes may be used to determine the absence or presence of the Y chromosome, or disomic copy numbers of the X chromosome.

### G. Early Detection of Causal Variants for Disease

Generally, the systems and methods of this disclosure may also be used to identify the presence of absence of causal variants, (i.e. SNPs, or CNVs) that may be useful in determining the risk or severity of a particular disease. For example, the systems and methods of this disclosure may be useful for patients with a familial history of Huntington's disease. This neurodegenerative disease is caused by variable length trinucleotide repeats in the Huntingtin gene (HTT). The length of this repeat may vary between individuals as well as between generations. The length of the repeat is thought to affect the severity of Huntington's disease itself. Determination of CNV levels may provide information regarding the number of repeats in the Huntingtin gene. This information may provide insight into the future severity of the disease in a fetus suspected of possessing the disease.

### XIV. Storage and Dissemination of Information

The information derived from analyzing loci of interest and identification of the presence or absence of genetic alterations can be communicated to any particular body, including the parents, guardian or owners of the fetus, from which the sample or sequence data is derived, clinician, research professional, medical professional, service provider, and medical insurer or insurance company. Medical professionals can be, for example, doctors, nurses, medical laboratory technologists, and pharmacists. Research professionals can be, for example, principle investigators, research technicians, postdoctoral trainees, and graduate students.

In some embodiments, a professional can be assisted by determining whether specific genetic alterations are present in a fetus, and communicate information about genetic alterations to a professional. After information about specific genetic alterations is reported, a medical professional can take one or more actions that can affect the parents. For example, a medical professional can record information in the parents' medical record regarding the embryo's risk of developing a developmental disorder. In some embodiments, a medical professional can record information regarding risk assessment of the embryo if it chosen to be implanted in a female.

In some embodiments, a medical professional can communicate information regarding an embryo's screening of developing a developmental disorder to a subject or a subject's family. In some embodiments, a medical professional can provide a family with information regarding a developmental disorder and risk assessment information, including treatment options, and referrals to specialists. In some embodiments, a medical professional can provide a copy of a subject's medical records to a specialist. In some embodiments, a research professional can apply information regarding a embryo's risk of developing a developmental disorder to advance scientific research.

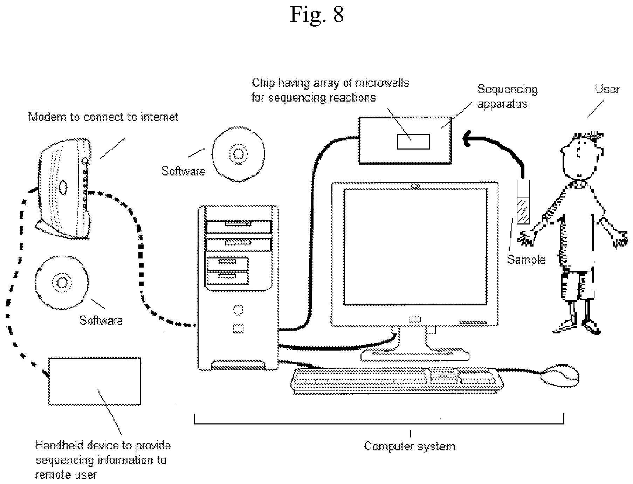

Any appropriate method can be used to communicate information to another person. For example, information can be given directly or indirectly to a professional and laboratory technician can input an embryo's genetic alteration as described herein into a computer-based record. In some embodiments, information is communicated by making a physical alteration to medical or research records. For example, a medical professional can make a permanent notation or flag a medical record for communicating the risk assessment to other medical professionals reviewing the record. In addition, any type of communication can be used to communicate the risk assessment information. For example, mail, e-mail, telephone, and face-to-face interactions can be used. The information also can be communicated to a professional by making that information electronically available to the professional. For example, the information can be communicated to a professional by placing the information on a computer database such that the professional can access the information as shown in Fig. 8. In addition, the information can be communicated to a hospital, clinic, or research facility serving as an agent for the professional. An exemplary diagram of computer based communication is shown in.

### XV. Examples

The following examples are included for illustrative purposes only and are not intended to limit the scope of the disclosure.

### Example 1: General Experimental Parameters for MIP Probe Ligation and Amplification

1. Probe design
2. Create a pooled stock at 100 attomole/ul/probe (60 million molecules/ul/probe)
3. Sample extraction (i.e isolation of cfDNA)
   A. Collect blood in Cell-Free DNA BCT using commericial kit
   B. For optimal results, include a Proteinase K treatment step (≥30 mAU/ml digest) at 60°C in the presence of chaotropic salts for 1 hour when extracting cell-free DNA and for 2 hours when extracting cellular genomic DNA.
4. Assay
   1. Combine:
      a. Extracted cfDNA
      b. with 1 ul of probe stock
      c. in IX ampligase buffer (Epicentre Technologies)
      d. to a volume of 7.8 ul
   2. Thermocycler:
      a. The annealing reaction mixture was heated to 95°C for 5 min.
      b. Then, the temperature was dropped one degree at a time for 1 min at each temperature, until 65°C was reached and
      c. Held at 65°C overnight.
   3. Add:
      a. 1 U ampligase enzyme (Epicentre Technologies)
      b. in IX ampligase buffer (Epicentre Technologies)
      c. total volume of 14.2 ul
   4. Thermocycler:
      a. 58°C for 2 min
   5. Add:
      a. 0.65 U of exonuclease I (Epicentre Technologies)
      b. 3.3 U of exonuclease III (Epicentre Technologies)
      c. with a negligible change in reaction mixture volume
   6. Thermocycler:
      a. 37°C for 15 min
      b. 80°C for 15 min to abolish the enzymatic activity
   7. We employed high fidelity polymerase (e.g. Kapa HiFi) and followed the manufacturer's instructions. HPLC-purified universal forward and reverse amplification primers from Integrated DNA Technologies:
      a. xxxxxxxxx-TACTGAGGTCGGTACACTCT
      b. yyyyyyyyyy-AGTAGCCGTGACTATCGACT
   8. Thermocycler:
      a. 95°C for 10 min
      b. 5 cycles of:
         i. 94°C for 30 s
         ii. 63°C for 30 s
         iii. 72°C for 30 s
      c. 72°C for 5 min

### Example 2: Detection of Trisomy 21

Peripheral blood samples are collected from a pregnant woman in her first or second trimester of pregnancy. Collected samples are centrifuged to obtain cell-free plasma. Cell-free DNA is the extracted from the plasma fraction using QiAmp DNA Blood Mini Kit (Qiagen) according to the manufacturer's instructions. Approximately 5 ng of DNA is obtained from 15 ml of blood.

MIP probe sets, designed for loci of interest in chromosome 21, are used to test the sample for detection of putative Trisomy 21. Loci of interest are selected throughout the chromosome, in regions of both arms and centromeric regions. Specific probe sequences are selected using optimization algorithms such as ROSO to select optimal probes and probe sets for hybridization. Selection criteria include site selectivity, minimization of cross reactivity with loci outside of chromosome 21, probe length, salt tolerance in hybridization reactions, minimization of secondary structures in the probes and minimization of probe-probe dimerization. Selection criteria and optimization are applied to individual probe sets as well as throughout the entirety of probes used. 100 probe sets are used to hybridize to chromosome 21 and each probe contains 2 probes.

Within each probe set, the first probe and second probe are selected such that no gap exists between the probes when bound to a complementary sequence. Chromosome 21 MIP probes are all commonly assigned an oligonucleotide barcode sequence ("X"). Barcode "X" in the MIP probes is flanked by 2 universal primer sites ("A") in a similar configuration as shown in Fig. 6B.

In addition to probes designed to hybridize to Chromosome 21, MIP probes designed towards a reference loci, outside of chromosome 21 (locus of interest) are used. 100 MIP probes, designed to hybridize throughout Chromosome 2 are used. Within each probe set, the first probe and second probe are selected such that no gap exists between the probes when bound to a complementary sequence, similar to probes for Chromosome 21. Selection criteria and optimization are applied to Chromosome 2 reference probes in a similar fashion as probes to Chromosome 21, as described herein. Chromosome 2 MIP probes are all commonly assigned an oligonucleotide barcode sequence ("Y"). Barcode "Y" in the MIP probes is flanked by 2 universal primer sites ("A") in a similar configuration as shown in Fig. 6B.

Both Chromosome 21 and 2 probe sets are simultaneously applied to the sample in a single hybridization reaction. Probe concentrations for both Chromosome 2 and 21 are added in excess of 5X the concentration of loci tested in the cell-free DNA sample. Sample polynucleotides are first denatured to produce single stranded cell-free DNA molecules at 95 C for 5 min and hybridization of probes is allowed to proceed overnight at 65 C. Ligation of bound MIP probes is then performed, with addition of NEB ligase and buffer. The reaction is placed at 25 C and the ligation is allowed to continue for 2 hours.

An exonuclease reaction is then performed to degrade unbound probes in the sample with the addition of a combination of ExoI/III (Epicentre) for 5 hours at 37 C. The reaction mixture is then run through a silica gel matrix as a clean-up step to remove degraded unbound probes and residual cell free DNA sample polynucleotides. Ligated products are eluted from the silica gel matrix and second strand synthesis is performed using KAPA/HIFI polymerase. A primer complementary to a portion of linker sequence in the probe used for second strand synthesis.

After second strand synthesis, a universal amplification step is performed using primers complementary to primer sites "A." Several rounds of linear amplification are performed followed by multiple rounds of logarithmic amplification cycles.

Amplification products are then ligated to universal adapters using Illumina Genomic Adaptor Oligo Mix and Illumina adaptors (Non-Index Y-Adaptors). The adaptor-ligated barcode sequences are purified from unligated adaptors, adaptor dimers, and other reagents using magnetic beads provided in the Agencourt AMPure XP PCR purification system. The purified amplified product is then eluted in 40 µl of Qiagen EB Buffer. The amplified DNA is sequenced using Illumina's Genome Analyzer II to obtain single-end reads. Reads for both barcodes X and Y are enumerated using Illumina software. A total set threshold of reads counts is set at 10000, after which counts are discontinued. A ratio of X/Y counts is calculated and compared additionally compared to a threshold reference value comprising a composite score of X/Y ratios obtained from similar experiments. Coefficient of variation and chi square analysis are performed to provide statistical significance of the calculated ratio. Determination of CNV is made by assessing the calculated ratio and comparison to a reference value.

### Example 3: Multi-CNV Test for Chromosomes 13, 18, 21, X, and Y

Using a similar experimental protocol strategy as described in Example 2, a multi chromosome test for CNV may be performed. Additionally, sub-chromosomal regions containing CNVs may also be detected. MIP probes, with similar characteristics to those as described in Example 2 are designed to hybridize various loci across Chromosome 13, 18, 21, X and Y. Reference probes are designed for the remaining chromosomes, Chromosomes 1-20 and 22. Individual probe sets are assigned a unique barcode sequence to resolve sequence counts for individual loci in regions of chromosomes. In addition, MIP probes are designed such that universal amplification sites flank unique barcode sequences that are additionally flanked by Illumina compatible adapter sequences. This design eliminates the need for an additional amplification step to incorporate adapter sequences for sequencing as described in Example 2.

Using similar biochemical and molecular biology steps as described in Example 2, ligation products for MIP probes hybridizing to loci across Chromosome 13, 18, 21, X and Y are isolated. Barcode -adapter sequence are amplified, identified and enumerated using Illumina platform sequencing in a similar fashion as described in Example 2. The total number barcodes for each chromosome (barcode density) is determined. Alternatively, the number of barcode sequences may be normalized to the length of the chromosome to generate a barcode density ratio. The normalization to chromosome length is not a required step, and can be performed solely to simplify the enumeration step.

The resulting barcode density for each chromosome is compared to the barcode density of each of the other chromosomes to derive a qualified chromosome "dose", which is calculated as the ratio of the barcode density for the chromosome of interest e.g. chromosome 21, and the barcode density of each of the remaining chromosomes (i.e. Chromosomes 1-20, 22 and X). Chromosomes doses are determined for all chromosomes in the sample. The chromosome dose for each of the chromosomes of interest provides a measure of the variation in the total number of barcodes for each chromosome of interest relative to that of each of the remaining chromosomes. The chromosome doses can identify the chromosome or a group of chromosomes i.e. normalizing chromosome that has a variation among samples that is closest to the variation of the chromosome of interest. CNV genetic alterations are determined based on comparison of chromosome dose ratios of the chromosomes of interest to normalized dose ratios for one or more remaining chromosomes.

### Example 4: Detection of SNP and CNV of Causal Variants for Autism

A test is performed to assess the risk of development of neurodevelopmental diseases such as autism in a fetal subject. In some cases of autism, the presence of certain SNPs and/CNV genetic alterations may predispose a subject to higher risk for development of the disease. In this test, a similar experimental protocol is used as described in Examples 2 and 3. In this test, however, MIP probe sets are designed to hybridize to loci of interest thought to be associated with the development of autism. Genetic alterations, such as rare SNPs and/or CNVs at one or more loci may indicate a higher or lower risk for the disease. Unique barcodes are assigned to individual probe sets for each locus. Loci are distributed across many chromosomes. Additionally, the 2 probes in each MIP probe set, are designed such that a gap exists between the probes when bound to a sample polynucleotide. The gap corresponds to a sequence in the sample polynucleotide in a particular locus which may contain an SNP related to the development of autism. Further, MIP probes are designed such that universal amplification priming sites flank the barcode sequence and the hybridization sequences as shown in Fig. 6A. Reference probes for loci outside the locus of interest may be designed to hybridize to various housekeeping genes and also include gaps between probes to capture SNPs of loci not associated with autism.

In an additional step to the experimental design as described in Examples 2 and 3, pre-ligation probes bound to sample polynucleotides are treated with a DNA polymerase, such as Klenow fragment to polymerize sequence in the gap between the two hybridized probes. This polymerized sequence may capture the identity of an SNP that exists in that region of the sequence.

After ligation and isolation of the products, amplification is performed. Different amplification steps may be performed. In one method, amplification is performed using primers designed for individual barcodes, or sequences common to one or more barcodes. These products are then sequenced and enumerated. Ratios of individual barcodes may be compared to determine CNV at one or more loci. In another step, another amplification reaction may be performed using a combination of a universal primer site, and a site designed adjacent to the location of the putative SNP. Amplification products contain both the barcode sequence and a portion of the hybridization sequence and sequence containing the SNP.

Sequences of these amplification products may be aligned to a reference sequence, using a portion of the hybridization sequence and the SNP to be identified. In other cases, alignment may not be used, as identification of the barcode will indicate the locus of interest. The barcode sequence and the SNP are both be enumerated. Comparisons of SNPs and barcodes are used to calculate allelic frequencies for both loci of interest and loci outside the loci of interest. Comparison of ratios may be used to determine specific causal variants (SNPs or CNVs) for autism and may be used in assessing a risk or risk factor for development of the disease in a fetal subject.

### Example 5: Detection of Genetic Alterations with High Sensitivity

A test for genetic alterations may also be performed in which amplification and sequencing steps may not be used. In this test, ligation products of bound MIPs may be generated as described herein, with a changes to downstream steps in the experimental protocol. After the ligation product is isolated, these products themselves may be used directly to enumerate sequences contained within the products. In some cases, the sample polynucleotide material may of sufficient amount to allow a sufficient number of probes to bind and be ligated, allowing barcodes and other sequences to be enumerated without an amplification step. In other methods, highly sensitive detection assay systems, such as the Nanostring nCounter system, may be used to enumerate barcodes directly from the ligation products, even when the number of bound probes to sample polynucleotides is relatively low.

The nCounter employs a hybridization based method using molecules that comprise a hybridization sequence and a corresponding fluorescent nanoparticle based barcode sequence. Together these elements provide the identity of the hybridization sequence as well as a quantifiable signal that may be used to enumerate the hybridization sequence bound to the ligation product present in a sample. In this test, after ligation products have been isolated, nCounter molecules, containing hybridization sequences designed for the barcode sequences contained in the ligation products may be used to enumerate the barcodes sequences. The nanoparticle barcodes in the nCounter molecules maybe also be assigned to specific hybridization sequences (polynucleotide barcodes), thus providing both identification and enumeration of a particular barcode assigned to a MIP probe. This test may be used for any application related to the systems and methods of the disclosure. Use of the nCounter system, or any related single molecule assay systems, may be used for the enumeration of sequences in the ligation products. This method may also be used in conjunction with various amplification or enrichment step of the ligation products.

### Example 6: Example Padlock-Style Probe

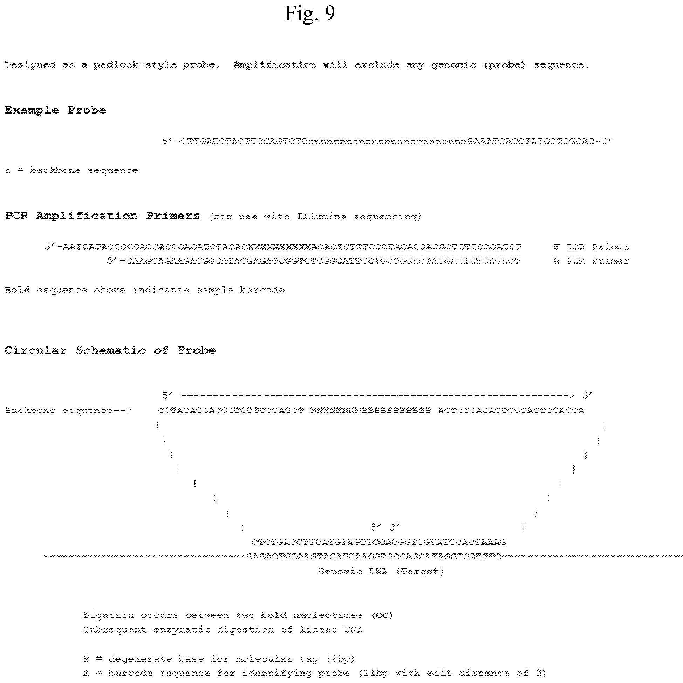

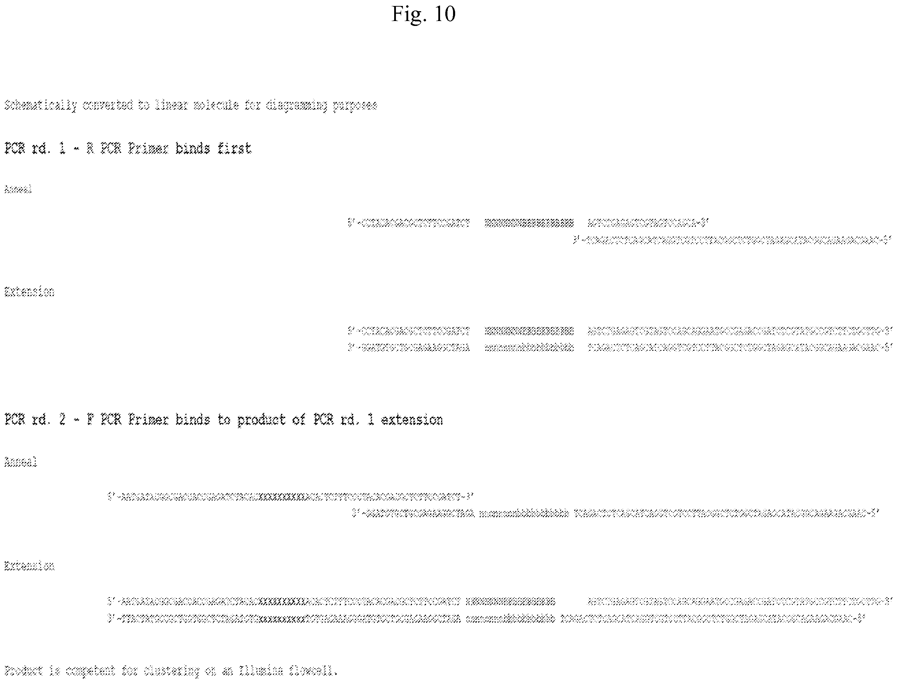

**Fig. 9** illustrates an example probe in accordance with the present disclosure, and example PCR amplification primers for amplifying a region of the probe. The "n" within the example probe represent backbone sequence, which is illustrated in more detail in the Circular Schematic, showing the probe bound to its target, with ends ligated to form a circular polynucleotide. In the Circular Schematic, "N" represents a degenerate based for a molecular tag, and "B" represents barcode sequence for identifying the probe. In the PCR amplification primers, an "X" represents sequence of a barcode associated with a particular sample. In this example, the 5' and 3' ends of the probe are ligated together without further extension. This is followed by PCR using the forward and reverse primers R PCR Primer and F PCR Primer, which is illustrated in **Fig. 10****.** R PCR primer hybridizes to the ligated probe, and is extended by a polymerase to generate a first extension product. F PCR Primer hybridizes to the first extension product, and is extended by a polymerase to generate a second extension product. Hybridization and extension of additional R PCR Primer and F PCR Primer may be repeated using the second extension product, and subsequent products, as templates to amplify the indicated probe sequence. The PCR product may then be sequenced using an Illumina flowcell.

## Claims

1. A method of testing for a genetic alteration at one or more loci in a sample comprising a mixture of maternal and fetal DNA polynucleotides, comprising the steps of:
a. obtaining maternal and fetal polynucleotides in a test sample;
b. hybridizing a plurality of molecular inversion probes (MIPs), each MIP comprising a first hybridization sequence, a second hybridization sequence, two or more universal priming sites, and an identifier, wherein the plurality of MIPs comprises a plurality of MIPs directed to at least one locus of interest and a plurality of MIPs directed to at least one reference locus outside the locus of interest in the sample comprising maternal and fetal polynucleotides;
c. optionally extending hybridization sequences using polymerase;
d. ligating MIPs to produce circular ligation products, wherein each of the circular ligation products is produced from one MIP;
e. isolating bound circular ligation products from unbound, linear MIPs;
f. amplifying a region from a ligation product using the two or more universal priming sites, wherein the region comprises the identifier;
g. enumerating the identifiers within the ligation products; and
h. determining the presence or absence of a genetic alteration at one or more loci, optionally comprising determining the sex of a fetus;
wherein the identifier is a molecular barcode;
wherein a first unique identifier is assigned to the MIPs targeting the at least one locus of interest and a second unique identifier is assigned to the MIPs targeting the at least one reference locus; and
wherein the first and second hybridization sequence of a MIP directed to at least one locus of interest bind sites on an identical strand of a polynucleotide sequence in the locus of interest.

2. The method of claim 1, wherein the MIPs further comprise a linker sequence.

3. The method of claim 1, wherein the barcode identifies the locus of interest, the MIP, or the sample origin.

4. The method of claim 1, wherein the locus of interest is a mitochondrial gDNA, chromosome, gene, exon, intron, intron-exon boundary, promoter, terminator, highly repetitive sequence, LTR, UTR, satellite sequences, centromere repeats, telomeres, noncoding sequences, coding sequences, regulators, transcription factor binding sites, ribosomal binding sites, or poly d(T) sequence epigenetic sequences, mobile elements, or transposons.

5. The method of claim 4, wherein the chromosome is selected from chromosome 1, chromosome 2, chromosome 3, chromosome 4, chromosome 5, chromosome, 6, chromosome, 7, chromosome 8, chromosome 9, chromosome, 10, chromosome 11, chromosome 12, chromosome 13, chromosome 14, chromosome 15, chromosome 16, chromosome 17, chromosome 18, chromosome 19, chromosome 20, chromosome 21, chromosome 22, X chromosome and Y chromosome.

6. The method of claim 1, wherein the reference locus is an internal reference comprising two or more chromosomes.

7. The method of claim 1, wherein the genetic alteration is a Copy Number Variant (CNV), a causal variant, a loss of heterozygosity (LOH), rearrangements, subtelomeric rearrangement, aneuploidy, partial aneuploidy, polyploidy, chromosomal instability, mutations, rare mutations, transversions, translocations, inversion, indels, deletions, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, abnormal changes in nucleic acid methylation, viral insertions, parasitic DNA insertions, or alterations in tandem repeats.

8. The method according to any of the preceding claims, further comprising using a bridging oligonucleotide that hybridizes to a region between two hybridization sequences.

9. The method according to any of the preceding claims, wherein amplifying is performed (a) through a universal amplification step, or (b) through a selective amplification step, or (c) on sequences not containing hybridization sequences.

10. The method according to any of the preceding claims, wherein isolating circular ligation products comprises degradation of unbound MIPs, optionally wherein degradation is performed using an exonuclease; or wherein isolating circular ligation products comprises affinity capture with a binding partner.

11. The method according to any of the preceding claims, further comprising determining SNP reads, CNV reads, or allele frequencies.

12. The method according to any of the preceding claims, wherein
a. at least one locus is tested for a genetic alteration;
b. at least 100 loci are tested for genetic alterations;
c. at least 500 loci are tested for genetic alterations;
d. at least 1000 loci are tested for genetic alterations;
e. at least one locus contains a polymorphism or putative polymorphism;
f. at least one locus is tested for copy number and is different than another locus containing a polymorphism;
g. the locus is a sub-chromosomal region; or
h. the locus is a single locus.

13. The method according to any one of the preceding claims, wherein
a. the MIPs and ligation products are artificial sequences;
b. the genetic alteration is fetal aneuploidy;
c. the method further comprises providing a medical decision based on determining the presence or absence of a genetic alteration;
d. the method further comprises providing a treatment recommendation based on determining the presence or absence of a genetic alteration;
e. enumerating is performed using statistical analysis, optionally wherein the statistical analysis is performed using a computer algorithm; or
f. enumerating is performed by a computer readable medium having processor-executable instructions.

14. The method of claim 1, the MIPs having a configuration of:
a. first hybridization sequence - universal priming site - identifier - universal priming site - second hybridization sequence;
b. first hybridization sequence - universal priming site - identifier - universal priming site - restriction site - second hybridization sequence;
c. first hybridization sequence - universal priming site - universal priming site - identifier - second hybridization sequence; or
d. first hybridization sequence - universal priming site - restriction site - universal priming site - identifier - second hybridization sequence.

15. The method of claim 1, wherein
a. a unique identifier is assigned for each unique set of hybridization sequences; or
b. identical identifiers are assigned for the same locus.

## Patentansprüche

1. Verfahren zum Testen auf eine genetische Veränderung an einem oder mehreren Loci in einer Probe, die eine Mischung aus mütterlichen und fetalen DNA-Polynukleotiden umfasst, umfassend die Schritte:
a. Erhalten von mütterlichen und fetalen Polynukleotiden in einer Testprobe;
b. Hybridisieren einer Vielzahl von molekularen Inversionssonden (molecular inversion probes - MIPs), wobei jede MIP eine erste Hybridisierungssequenz, eine zweite Hybridisierungssequenz, zwei oder mehr universelle Priming-Stellen und einen Identifikator umfasst, wobei die Vielzahl von MIPs eine Vielzahl von MIPs, die auf mindestens einen Locus von Interesse gerichtet sind, und eine Vielzahl von MIPs, die auf mindestens einen Referenz-Locus außerhalb des Locus von Interesse gerichtet sind, umfasst, in der Probe, die mütterliche und fetale Polynukleotide umfasst;
c. optional Verlängern von Hybridisierungssequenzen unter Verwendung von Polymerase;
d. Ligieren von MIPs, um kreisförmige Ligationsprodukte herzustellen, wobei jedes der kreisförmigen Ligationsprodukte aus einer MIP produziert wird;
e. Isolieren gebundener kreisförmiger Ligationsprodukte von ungebundenen, linearen MIPs;
f. Amplifizieren einer Region von einem Ligationsprodukt unter Verwendung der zwei oder mehr universellen Priming-Stellen, wobei die Region den Identifikator umfasst;
g. Zählen der Identifikatoren innerhalb der Ligationsprodukte; und
h. Bestimmen des Vorliegens oder Nichtvorliegens einer genetischen Veränderung an einem oder mehreren Loci, optional umfassend Bestimmen des Geschlechts eines Fetus;
wobei der Identifikator ein molekularer Strichcode ist;
wobei ein erster eindeutiger Identifikator den MIPs, die auf den mindestens einen Locus von Interesse abzielen, zugewiesen wird und ein zweiter eindeutiger Identifikator den MIPs, die auf den mindestens einen Referenz-Locus abzielen, zugewiesen wird; und
wobei die erste und die zweite Hybridisierungssequenz einer MIP, die auf mindestens einen Locus von Interesse gerichtet ist, Stellen auf einem identischen Strang einer Polynukleotidsequenz in dem Locus von Interesse binden.

2. Verfahren nach Anspruch 1, wobei die MIPs ferner eine Linkersequenz umfassen.

3. Verfahren nach Anspruch 1, wobei der Strichcode den Locus von Interesse, die MIP oder die Probenherkunft identifiziert.

4. Verfahren nach Anspruch 1, wobei der Locus von Interesse eine mitochondriale gDNA, ein Chromosom, ein Gen, ein Exon, ein Intron, eine Intron-Exon-Grenze, ein Promotor, ein Terminator, eine stark repetitive Sequenz, LTR, UTR, Satellitensequenzen, Zentromer-Repeats, Telomere, nichtkodierende Sequenzen, kodierende Sequenzen, Regulatoren, Transkriptionsfaktor-Bindungsstellen, ribosomale Bindungsstellen oder epigenetische Sequenzen der Poly-d(T)-Sequenz, mobile Elemente oder Transposons ist.

5. Verfahren nach Anspruch 4, wobei das Chromosom ausgewählt ist aus Chromosom 1, Chromosom 2, Chromosom 3, Chromosom 4, Chromosom 5, Chromosom, 6, Chromosom, 7, Chromosom 8, Chromosom 9, Chromosom, 10, Chromosom 11, Chromosom 12, Chromosom 13, Chromosom 14, Chromosom 15, Chromosom 16, Chromosom 17, Chromosom 18, Chromosom 19, Chromosom 20, Chromosom 21, Chromosom 22, X-Chromosom und Y-Chromosom.

6. Verfahren nach Anspruch 1, wobei der Referenz-Locus eine interne Referenz ist, die zwei oder mehr Chromosomen umfasst.

7. Verfahren nach Anspruch 1, wobei die genetische Veränderung eine Copy Number Variant (CNV), eine kausale Variante, ein Verlust der Heterozygotie (loss of heterozygosity - LOH), Umlagerungen, subtelomere Umlagerung, Aneuploidie, partielle Aneuploidie, Polyploidie, chromosomale Instabilität, Mutationen, seltene Mutationen, Transversionen, Translokationen, Inversion, Indels, Deletionen, Chromosomenstrukturveränderungen, Genfusionen, Chromosomenfusionen, Gentrunkierungen, Genamplifikation, Genduplikationen, Chromosomenläsionen, DNA-Läsionen, anormale Veränderungen in chemischen Modifikationen von Nukleinsäuren, anormale Veränderungen in epigenetischen Mustern, anormale Veränderungen in der Nukleinsäuremethylierung, virale Insertionen, parasitäre DNA-Insertionen oder Veränderungen in Tandem-Repeats ist.

8. Verfahren nach einem der vorhergehenden Ansprüche, ferner umfassend Verwenden eines Brücken-Oligonukleotids, das mit einer Region zwischen zwei Hybridisierungssequenzen hybridisiert.

9. Verfahren nach einem der vorhergehenden Ansprüche, wobei das Amplifizieren (a) durch einen universellen Amplifikationsschritt oder (b) durch einen selektiven Amplifikationsschritt oder (c) an Sequenzen durchgeführt wird, die keine Hybridisierungssequenzen enthalten.

10. Verfahren nach einem der vorangehenden Ansprüche, wobei das Isolieren von kreisförmigen Ligationsprodukten einen Abbau von ungebundenen MIPs umfasst, optional wobei der Abbau unter Verwendung einer Exonuklease durchgeführt wird; oder wobei das Isolieren von kreisförmigen Ligationsprodukten eine Affinitätsbindung mit einem Bindungspartner umfasst.

11. Verfahren nach einem der vorhergehenden Ansprüche, ferner umfassend Bestimmen von SNP-Reads, CNV-Reads oder Allelhäufigkeiten.

12. Verfahren nach einem der vorhergehenden Ansprüche, wobei
a. mindestens ein Locus auf eine genetische Veränderung getestet wird;
b. mindestens 100 Loci auf genetische Veränderungen getestet werden;
c. mindestens 500 Loci auf genetische Veränderungen getestet werden;
d. mindestens 1000 Loci auf genetische Veränderungen getestet werden;
e. mindestens ein Locus einen Polymorphismus oder mutmaßlichen Polymorphismus enthält;
f. mindestens ein Locus auf Kopienzahl getestet wird und sich von einem anderen Locus, der einen Polymorphismus enthält, unterscheidet;
g. der Locus eine subchromosomale Region ist; oder
h. der Locus ein einzelner Locus ist.

13. Verfahren nach einem der vorhergehenden Ansprüche, wobei
a. die MIPs und Ligationsprodukte künstliche Sequenzen sind;
b. die genetische Veränderung fetale Aneuploidie ist;
c. das Verfahren ferner Bereitstellen einer medizinischen Entscheidung basierend auf dem Bestimmen des Vorliegens oder Nichtvorliegens einer genetischen Veränderung umfasst;
d. das Verfahren ferner Bereitstellen einer Behandlungsempfehlung basierend auf dem Bestimmen des Vorliegens oder Nichtvorliegens einer genetischen Veränderung umfasst;
e. das Zählen unter Verwendung einer statistischen Analyse durchgeführt wird, optional wobei die statistische Analyse unter Verwendung eines Computeralgorithmus durchgeführt wird; oder
f. das Zählen durch ein computerlesbares Medium durchgeführt wird, das prozessorausführbare Anweisungen aufweist.

14. Verfahren nach Anspruch 1, wobei die MIPs eine Konfiguration aufweisen von:
a. erste Hybridisierungssequenz - universelle Priming-Stelle - Identifikator - universelle Priming-Stelle - zweite Hybridisierungssequenz;
b. erste Hybridisierungssequenz - universelle Priming-Stelle - Identifikator - universelle Priming-Stelle - Restriktionsstelle - zweite Hybridisierungssequenz;
c. erste Hybridisierungssequenz - universelle Priming-Stelle - universelle Priming-Stelle - Identifikator- zweite Hybridisierungssequenz; oder
d. erste Hybridisierungssequenz - universelle Priming-Stelle - Restriktionsstelle - universelle Priming-Stelle - Identifikator- zweite Hybridisierungssequenz.

15. Verfahren nach Anspruch 1, wobei
a. ein eindeutiger Identifikator jedem eindeutigen Satz von Hybridisierungssequenzen zugewiesen wird; oder
b. identische Identifikatoren demselben Locus zugewiesen werden.

## Revendications

1. Procédé permettant de tester une modification génétique au niveau d'un ou plusieurs locus dans un échantillon comprenant un mélange de polynucléotides d'ADN maternels et fœtaux, comprenant les étapes de :
a. obtention de polynucléotides maternels et fœtaux dans un échantillon d'essai ;
b. hybridation d'une pluralité de sondes d'inversion moléculaire (MIP), chaque MIP comprenant une première séquence d'hybridation, une seconde séquence d'hybridation, deux sites d'amorçage universels ou plus, et un identifiant, ladite pluralité de MIP comprenant une pluralité de MIP dirigée vers au moins un locus d'intérêt et une pluralité de MIP dirigée vers au moins un locus de référence en dehors du locus d'intérêt dans l'échantillon comprenant des polynucléotides maternels et fœtaux ;
c. extension éventuelle de séquences d'hybridation à l'aide de la polymérase ;
d. ligature de MIP pour produire des produits de ligature circulaires, chacun des produits de ligature circulaires étant produit à partir d'une MIP ;
e. isolement des produits de ligature circulaires liés des MIP linéaires non liées ;
f. amplification d'une région à partir d'un produit de ligature à l'aide des deux sites d'amorçage universels ou plus, ladite région comprenant l'identifiant ;
g. énumération des identifiants au sein des produits de ligature ; et
h. détermination de la présence ou de l'absence d'une modification génétique au niveau d'un ou plusieurs locus, comprenant éventuellement la détermination du sexe d'un foetus ;
ledit identifiant étant un code à barres moléculaire ;
un premier identifiant unique étant attribué aux MIP ciblant ledit au moins un locus d'intérêt et un second identifiant unique étant attribué aux MIP ciblant ledit au moins un locus de référence ; et
lesdites première et seconde séquences d'hybridation d'une MIP dirigée vers au moins un locus d'intérêt se liant à des sites sur un brin identique d'une séquence polynucléotidique dans le locus d'intérêt.

2. Procédé selon la revendication 1, lesdites MIP comprenant en outre une séquence de liaison.

3. Procédé selon la revendication 1, ledit code à barres identifiant le locus d'intérêt, la MIP ou l'origine de l'échantillon.

4. Procédé selon la revendication 1, ledit locus d'intérêt étant un ADNg mitochondrial, un chromosome, un gène, un exon, un intron, une limite intron-exon, un promoteur, un terminateur, une séquence hautement répétitive, une LTR, une UTR, des séquences satellites, des répétitions de centromères, des télomères, des séquences non codantes, des séquences codantes, des régulateurs, des sites de liaison de facteur de transcription, des sites de liaison ribosomique ou des séquences épigénétiques de séquence poly d(T), des éléments mobiles ou des transposons.

5. Procédé selon la revendication 4, ledit chromosome étant choisi parmi le chromosome 1, le chromosome 2, le chromosome 3, le chromosome 4, le chromosome 5, le chromosome 6, le chromosome 7, le chromosome 8, le chromosome 9, le chromosome 10, le chromosome 11, le chromosome 12 , le chromosome 13, le chromosome 14, le chromosome 15, le chromosome 16, le chromosome 17, le chromosome 18, le chromosome 19, le chromosome 20, le chromosome 21, le chromosome 22, le chromosome X et le chromosome Y.

6. Procédé selon la revendication 1, ledit locus de référence étant une référence interne comprenant deux chromosomes ou plus.

7. Procédé selon la revendication 1, ladite modification génétique étant une variante du nombre de copies (CNV), une variante causale, une perte d'hétérozygotie (LOH), des réarrangements, un réarrangement subtélomérique, une aneuploïdie, une aneuploïdie partielle, une polyploïdie, une instabilité chromosomique, des mutations, des mutations rares, des transversions, des translocations, une inversion, des indels, des délétions, des modifications de la structure chromosomique, des fusions de gènes, des fusions de chromosomes, des troncatures de gènes, une amplification de gène, des duplications de gènes, des lésions chromosomiques, des lésions de l'ADN, des changements anormaux dans des modifications chimiques d'acides nucléiques, des changements anormaux dans des motifs épigénétiques, des changements anormaux dans la méthylation d'acides nucléiques, des insertions virales, des insertions d'ADN parasites ou des modifications dans des répétitions en tandem.

8. Procédé selon l'une quelconque des revendications précédentes, comprenant en outre l'utilisation d'un oligonucléotide de pontage qui s'hybride à une région située entre deux séquences d'hybridation.

9. Procédé selon l'une quelconque des revendications précédentes, ladite amplification étant réalisée (a) par une étape d'amplification universelle, ou (b) par une étape d'amplification sélective, ou (c) sur des séquences ne contenant pas de séquences d'hybridation.

10. Procédé selon l'une quelconque des revendications précédentes, ledit isolement des produits de ligature circulaires comprenant la dégradation des MIP non liées, éventuellement ladite dégradation étant effectuée à l'aide d'une exonucléase ; ou ledit isolement des produits de ligature circulaires comprenant la capture par affinité avec un partenaire de liaison.

11. Procédé selon l'une quelconque des revendications précédentes, comprenant en outre la détermination des lectures de SNP, des lectures de CNV ou des fréquences d'allèles.

12. Procédé selon l'une quelconque des revendications précédentes,
a. au moins un locus étant testé pour une modification génétique ;
b. au moins 100 locus étant testés pour des modifications génétiques ;
c. au moins 500 locus étant testés pour des modifications génétiques ;
d. au moins 1000 locus étant testés pour des modifications génétiques ;
e. au moins un locus contenant un polymorphisme ou un polymorphisme putatif ;
f. au moins un locus étant testé pour le nombre de copies et étant différent d'un autre locus contenant un polymorphisme ;
g. ledit locus étant une région sous-chromosomique ; ou
h. ledit locus étant un locus unique.

13. Procédé selon l'une quelconque des revendications précédentes,
a. lesdites MIP et lesdits produits de ligature étant des séquences artificielles ;
b. ladite modification génétique étant une aneuploïdie foetale ;
c. ledit procédé comprenant en outre la fourniture d'une décision médicale basée sur la détermination de la présence ou de l'absence d'une modification génétique ;
d. ledit procédé comprenant en outre la fourniture d'une recommandation de traitement basée sur la détermination de la présence ou de l'absence d'une modification génétique ;
e. l'énumération étant effectuée à l'aide d'une analyse statistique, éventuellement ladite analyse statistique étant effectuée à l'aide d'un algorithme informatique ; ou
f. ladite énumération étant effectuée par un support lisible par ordinateur comportant des instructions exécutables par processeur.

14. Procédé selon la revendication 1, les MIP présentant une configuration de :
a. première séquence d'hybridation - site d'amorçage universel - identifiant - site d'amorçage universel - seconde séquence d'hybridation ;
b. première séquence d'hybridation - site d'amorçage universel - identifiant - site d'amorçage universel - site de restriction - seconde séquence d'hybridation ;
c. première séquence d'hybridation - site d'amorçage universel - site d'amorçage universel - identifiant - seconde séquence d'hybridation ; ou
d. première séquence d'hybridation - site d'amorçage universel - site de restriction - site d'amorçage universel - identifiant - seconde séquence d'hybridation.

15. Procédé selon la revendication 1,
a. un identifiant unique étant attribué pour chaque ensemble unique de séquences d'hybridation ; ou
b. des identifiants identiques étant attribués pour le même locus.
